# EUROPEAN PATENT SPECIFICATION

(11) **EP 1 352 249 B1**
(45) Date of publication and mention of the grant of the patent: **24.05.2006**
(21) Application number: 02729518.7
(22) Date of filing: 09.01.2002
(51) Int. Cl.: G01N 33/68, G06F 19/00, G01N 33/567, G01N 33/543

(54) **PROTEOME ANALYSIS METHOD**
PROTEOM-ANALYSEVERFAHREN
PROCEDE D'ANALYSE PROTEOMIQUE

(30) Priority: 09.01.2001 US 260433 P; 02.03.2001 US 272981 P; 03.01.2002 US 38918
(43) Date of publication of application: 15.10.2003
(73) Proprietor: Protosera Inc., Osaka 573-1153 (JP)
(72) Inventor: TANAKA, Kenji, c/o Mitsubishi Pharma Corp., Tokyo 103-8405 (JP); LEE, Lyang-ja, c/o Mitsubishi Pharma Corp., Tokyo 103-8405 (JP); MUKAI, Hiromichi, c/o Mitsubishi Pharma Corp., Tokyo 103-8405 (JP); MUNECHIKA, Koji, c/o Mitsubishi Pharma Corp., Tokyo 103-8405 (JP); ARIKUNI, Hisashi, Miura-gun, Kanagawa 240-0112 (JP); SHIWA, Mieko, c/o Ciphergen Biosystems, K.K.,, Yokohama-shi, Kanagawa 240-0005 (JP); OCHIAI, Bungo,, Yonezawa-shi, Yamagata 992-0055 (JP)
(74) Representative: Vossius & Partner
(86) International application number: PCT/JP2002/000029
(87) International publication number: WO 2002/056026

(56) References cited:
- WO-A2-00/43791
- DE-A- 4 017 805
- GB-A- 2 312 782
- US-A- 5 585 275
- HERMANN THOMAS ET AL: "Proteome analysis of Corynebacterium glutamicum." ELECTROPHORESIS, vol. 22, no. 9, May 2001 (2001-05), pages 1712-1723, XP008004268 ISSN: 0173-0835
- SIMPSON RICHARD J ET AL: "Proteomic analysis of the human colon carcinoma cell line (LIM 1215): Development of a membrane protein database." ELECTROPHORESIS, vol. 21, no. 9, May 2000 (2000-05), pages 1707-1732, XP002201607 ISSN: 0173-0835
- ZHU H ET AL: "Global analysis of protein activities using proteome chips." SCIENCE. UNITED STATES 14 SEP 2001, vol. 293, no. 5537, 14 September 2001 (2001-09-14), pages 2101-2105, XP002201608 ISSN: 0036-8075
- BIOCHEMISTRY vol. 37, no. 2, 1998, pages 507 - 522
- OSHRY L. ET AL BIOCHIM. BIOPHYS. ACTA vol. 1066, 1991, pages 239 - 244
- CHEN P. ET AL THE JOURNAL OF BIOLOGICAL CHEMISTRY vol. 276, no. 24, June 2001, pages 21098 - 21104
- PARMAR M.M. ET AL BIOCHIM. BIOPHYS. ACTA vol. 1421, pages 77 - 90

## Description

### TECHNICAL FIELD OF THE INVENTION

The present invention relates to the methodology, techniques and devices for functional proteomics that enables collective finding and collective quantification of membrane proteins and their ligands, as well as their functional (interaction) analysis. The present invention also relates to a novel pharmacoproteomic analysis method of a disease using the membrane proteins and their ligands as indices, which method comprises finding, isolating, identifying and quantifying membrane proteins and their ligands, that are involved in the onset, exacerbation and cure of particular diseases, elucidating their functions and constructing a database of their kinds and quantification values, and to the database itself. The present invention further relates to a method of constructing membrane protein library of every organism, and also relates to isolation methods of a membrane protein, which methods being capable of retaining the structure and function of the protein and being essential for the study of membrane proteins, identification methods, quantification methods, and other basic methods necessary for functional elucidation of membrane proteins.

### BACKGROUND OF THE INVENTION

With the advance in the fundamental researches for drug discovery, including molecular biology and genomics (genome science), the landscape of drug discovery in these several years has been rapidly changing and novel methods for drug discovery, represented by genomic drug discovery, are being developed.

The discovery of a novel medicine rests on the finding of a substance that has an particular physiological activity in certain disease(s). The substances that have such physiological activities are mostly proteins, and elucidation of the structure and function of proteins is the essential problem in the development of medicines.

For biogenic activities from fertilization to development, differentiation, growth, metabolism and to death, proteins embedded in membranes carry out important functions. These membrane proteins function as a membrane receptor to transmit extracellular information to the inside of the cell, as a specific membrane transporter of physiological substances from the inside to the outside of the cell and vice versa, and as lining proteins of membranes that support dynamic membrane structures. The difficulties in purification, isolation and in functional analyses have delayed the researches of membrane-associated proteins.

Because the function (physiological action) of a protein cannot be predicted from the nucleic acid sequence determined by genomics, establishment of a method for connecting the genetic information to a new drug is demanded for post-genomics. One of them drawing attention is proteomics (protein science). Proteomics aims at the isolation, identification and clarification of the function of all proteins existing more than 100,000. As the situation stands, how to connect the genomic information to the understanding of protein functions is the strategic goal of the drug discovery in the 21st century.

The high diversity of protein molecular structures and functions involved in the biogenic activities in general is incomparable with the diversity of DNA molecules. In this sense, applying the strategy of genomics, which achieved a success by applying the only DNA sequencing method to structurally similar 24 human chromosomes, directly to proteomics that deals with the objects affluent in diversity is impractical. Only with the DNA sequence, prediction of biogenic activity is impossible. Thus, proteomics capable of elucidation of protein functions is awaited.

The relationship between molecular structure and molecular activity is a fundamental in the study of biology. The structure-activity relationship is critical for the understanding of any biological reaction, such as enzyme functions, method of intercellular communication, and cellular regulation and feedback system.

Protein is vital to life phenomena and exhibits its function in the interaction with other molecules including a protein molecule, a DNA molecule, a synthetic compound or a photon and so on. Understanding a certain protein goes beyond mere recitation of the physical or chemical properties of this molecule. It includes finding what interaction occurs with which molecule, by identifying the molecules influencing each other and elucidating the mode of phenomena of the interaction (physiological action).

Certain species of macromolecules are known to interact and bind with other molecules, having highly specific 3-dimensional and electron distribution. Any macromolecule having such specificity is considered to be a receptor, whether it is an enzyme that catalyzes hydrolysis of metabolic intermediates, a cell surface protein that mediates membrane transport of ions, a glycoprotein useful for identifying a particular cell from neighboring cells, an IgG antibody circulating in plasma, an oligonucleotide sequence of nuclear DNA or something else. Various molecules that a receptor selectively binds with are known as ligands.

About half of the existing pharmaceutical products are known to act via a receptor on a cell membrane. Therefore, elucidation of a novel membrane protein and its physiological ligand provides a revolutionary screening system for the development of novel therapeutic agents for various diseases. Moreover, construction of a database of new and known membrane proteins and ligands involved in diseases enables elucidation of molecular dynamics in the diseases, where pharmacogenomics cannot reach, and is expected to lead to the development of novel diagnostic methods and novel therapeutic agents.

Many methods are available to discover unknown receptors and ligands, but the number of receptors or ligands obtainable from conventional ideas, methods and experiments is sometimes limited by their characteristics. Discovery of a complex type receptor consisting of plural peptides is associated with still more difficult problems. Novel receptors and ligands are found by novel technologies; such as X-ray crystal diffraction or genetic recombination techniques. However, such new methods depend on accidental coincidence and need a long period of biochemical research, or are applicable to extremely limited species of molecular.

Given the consideration set out in the above, the study of membrane proteins such as membrane receptors and membrane transporters as the targets of proteomics for the elucidation of a part of the physiological function thereof (=identification of physiological ligand) is of greater significance.

GB-A1 23 12 782 describes prefabricated MALDI layers suitable for the analysis of analyte substances. Said layers comprise at least one ionizing component for ionizing the analyte and a protecting compound for protecting the ionizing component.

Parmar et al., Biochimica et Biophysica Aeta 1421, 77-90 (1999) describes the incorporation of bacterial membrane proteins into liposomes to study factors influencing protein reconstruction.

Ostry, et al., Biocimica et Biophysica Aeta 1066, 239-244 (1991) describes annexia-mediated membrane fusion of human neutrophil plasma membranes and phospholipid residues

Simpson et al; Electrophoresis 21, 1707-1732 (2000) describes a proteome analysis of the human colon carcinoma cel line (LIM1215) by employing a non-2-DE method for identifying membrane proteins. It is described that proteins from an enriched membrane preparation which were initially fractionated are in-gel digested so as to be further analyzed by ESI-IT-MS.

DE-A1 40 17 805 describes means and methods for providing an analyte for testing wherein molecules of said analyte are bound in an essentially 2-dimensional layer characterized in that said molecules of said analyte are desorbed from the surface by laser desorption. It is described that' the methods improve in particular the desorption of molecules of the analyte from the surface of a probe to be tested.

Hermann et al., Electrophoresis 22, 1712-1723 (2001) disclose a proteome analysis method for analysing both membrane protein and water-soluble protein fractions from biological samples separately, but not the mutual interaction thereof.

Conventional study of proteomics has exclusively relied on the two-dimensional electrophoresis method as a means of separating proteins. However, the current method has the following five problems, when analyzing total proteins of a certain cell.

Firstly, when the entire biological sample is electrophoresed on a single gel, the analysis per se is ruined because proteins having a high molecular weight and insoluble membrane proteins remain near the origin without migrating. Thus, conventional two-dimensional electrophoresis cannot afford analysis of total proteins that express in a cell, and has been used for the analysis of specific proteins (mostly soluble, low molecular weight proteins). The biology in the 20^{th} century has made drastic advancements due to the development of molecular biological techniques, but most of the targeted proteins were water-soluble proteins. The proteomic study in early stages revealed that the total number of proteins detected in plasma was about 200, but the number of proteins contained in cell homogenate was 1400 - 4000. The total number of proteins, expressed from about 30000 genes encoding human protein, is expected to be more than 100,000. This means only several percent of the total proteins can be detected even by the most sensitive proteomics technology.

Any life phenomenon can be explained as a function of protein, where life is born by dividing self and non-self with a membrane. The task of recognizing non-self in the outer world and making the self respond thereto is performed by membrane proteins. Nevertheless, most of the undetectable proteins by the current proteomics technology are these membrane proteins playing an important role in the life activities, which show function upon being associated with a membrane or embedded in a membrane.

Secondly, a protein complex consisting of plural proteins and exerting a unique function in a cell does not allow analysis of the structure (quaternary structure of protein) and function actually present in the body, because the bindings between proteins based on hydrophobic interaction are dissociated when electrophoresed in a buffer containing a detergent.

Thirdly, effective idea, method or a technique for grouping the total proteins contained in a biological sample has not been provided. The number of total proteins expressed from human genes totaling to about 30,000 in number reaches a large number exceeding 100,000. They are subject to splicing after transcription from the same gene, thereby producing proteins having shorter peptide chain than others, and to various modifications by sugar, lipid, phosphate group and the like, after translation. As a result, proteins, the target of proteomics, consist of far more complicated molecule groups than the DNA polymer molecule, the target of genomics. Based on these facts, a hypothesis is set up that the only methodology (sequence determination for nucleic acid) based on the only purpose (to determine the nucleic acid sequence) can not elucidate diverse structures and functions of proteome. It is thus very important to group the proteins contained in a biological sample based on some idea before proteome analysis and some attempts at pretreatment has been made up to this day. For example, Molly et al. [Eur. J. Biochem. 267, 2871-2881 (2000)] and Santoni et al. [Electrophoresis 21, 1054-1070 (2000)] pretreated a sample with strong solubilizer, but have not solubilized all proteins. Herbert et al. [Electrophoresis 21, 3639-3648 (2000)] and Zuo et al. [Anal. Biochem. 284, 266-278 (2000)] pretreated samples by separating depending on their isoelectric point, but it is difficult to set appropriate range of isoelectric point for target proteins, and isoelectric focusing was prevented. It should be noted that these attempts are aiming at partial improvement of electrophoresis method, and not aiming at total proteome analysis of by grouping total proteome realized by this invention. To date, however, since no effective idea of grouping has been proposed, the same methods are employed from sample preparation to the analysis thereafter, without grouping samples. This forces the proteomic study to encounter the above-mentioned two problems.

Fourthly, in the conventional study of proteomics, time-consuming, multi-step complicated manipulation of segmenting the gel into small fragments and extracting the protein from each fragment using a particular solution is required before MS analysis. Complicated manipulations of this method refuse miniaturization of devices, shortening of measurement time, processing of multiple samples, or automation of entire device.

Fifth problem is the existence of many kinds of the so-called "low-abundance protein". Only 100 genes code 50 weight percent of all proteins in Yeast, and this means the another 50 % of proteins are the product of several thousands genes. A lot of most important proteins such as regulatory proteins or signal-transduction-related proteins including receptors are included among the "low-abundance proteins", so the current proteome analysis methods based on electrophoresis cannot analyze them.

To solve such limited ability of electrophoresis and to elucidate protein-protein interaction, many attempts have been made, such as ICAT (istope-coded affinity tag) method [Gygi et al. Nat. Biotech. 10, 994-999 (1999)], two-hybrid system in yeast, BIA-MS-MS, protein array method (solution or chip) [Zhou et al. TRENDS in Biotechnology 19, S34-S391 or peptide mix of LC-MS-MS. However, these novel methods and technologies have not realized expression analysis, interaction analysis and network analysis of total proteome that are ultimate purpose of proteomics. Even protein array methods, which is the most promising among the above-mentioned methods, including solid phase protein array method (chip method) [Fung et al. Curr. Opin. Biotechnol. 12, 65-69 (2001)] and liquid phase protein array method (e.g., fluorescence-encoded beads [Fulton et al. Clin. Chem. 43, 1749-1756 (1997)] [Han et al. Nat. Bioptechnol. (in press)] cannot realize two most important basic technologies i.e., purification and separation of total proteome and selective immobilization of total proteome onto a support.

It is therefore an object of the present invention to provide an idea, methods and techniques that solve all the problems currently facing the proteome studies, thereby to make possible for the first time ever the proteomic study of membrane proteins, which has been, of all proteins, recognized to be crucial for the elucidation of biological function and diseases but has got far behind in the study due to the greatest difficulty of purification and isolation while retaining its function. More specifically, the present invention focuses the proteomic study on that of membrane proteins and aims at isolating the membrane protein intact, constructing a membrane protein library wherein the membrane proteins retain their native structure and function, and explaining the function of the membrane protein in terms of the interaction with its ligand (i.e., finding and identifying a native effector molecule of membrane protein, or ligand), and for this end, providing a basic idea, methods, techniques and devices permitting analysis of any membrane protein. Furthermore, the present invention aims at pooling the thus-obtained information of membrane proteins and their physiological ligands as a database for use in elucidating the mechanism of diseases, in which they are involved, and further opening a window to establish novel diagnostic methods of such diseases and to develop novel therapeutic agents.

### SUMMARY OF THE INVENTION

The present invention provides a basic idea no one has ever proposed in this field that suggests grouping of the total proteins constituting the body into membrane proteins and other water-soluble proteins and analysing their mutual interaction for the proteomic study of membrane protein. The present invention is based on this idea of grouping, taking note of the native local site where protein exhibits its function.

Such insight and introduction of grouping wherein only the water-soluble proteins are electrophoresed and membrane proteins are analyzed by a new method (i.e., membrane protein is embedded in an artificial liposome that models on the cell membrane lipid bilayer and membrane protein library is constructed) resolve difficulty in the membrane protein analysis by electrophoresis, and establish the basic principle of analyzing the function of membrane protein (interaction with water-soluble protein) through analysis of total proteins (membrane protein and water-soluble protein), utilizing the biological affinity for water-soluble protein after the grouping. This essential technology is also applicable to screening and analysis of membrane protein-membrane protein or water-soluble protein-water-soluble protein interaction, providing an innovative theory to the future proteomics.

More particularly, the present invention provides a proteome analysis method, characterized by grouping the total proteins in the body into membrane proteins and water-soluble proteins, and collectively analyzing membrane proteins and their ligands based on the biological affinity between them, as well as various devices therefor. The method comprises the following steps:
(1) isolating a water-soluble protein fraction from a biological sample, separating water-soluble proteins in the fraction by gel electrophoresis, bringing the gel after electrophoresis into contact with a ligand support having a surface that can immobilize the proteins retaining the physiological function, and transferring the water-soluble proteins in the gel onto the ligand support;
(2) isolating a membrane fraction from a cell sample and fusing the membrane fraction with liposomes to prepare a membrane protein library (a set of membrane protein-embedded liposomes) wherein all membrane proteins are attached to or penetrated into its lipid bilayer;
(3) bringing the water-soluble proteins immobilized on the ligand support in contact with the membrane protein library to trap membrane proteins having affinity to the water-soluble proteins on the ligand support; and
(4) analyzing both or either of the membrane proteins and the water-soluble proteins having affinity by a means capable of analyzing at least one of the physical or chemical properties of those proteins.

Preferably, the means capable of analyzing at least one of the physical or chemical properties of protein is mass spectrometric analysis, and therefore, a suitable ligand support is a plate for mass spectrometry.

The present invention specifically solves the problems as follows.

The secondary, tertiary and quaternary structures and physiological function of the membrane protein can be retained, because the membrane protein is transferred to an artificial liposome membrane keeping the biological conditions of its hydrophobic regions and hydrophilic regions, without using a protein denaturing agent, a protein solubilizer or any other treatment condition that deviates the physiological conditions under which membrane' proteins exist. As regards the receptor, any structure and function of any biomembrane-type receptors, inclusive of GPI type, GPCR type, and oligomer type receptors, can be retained. Therefore, every membrane protein can be prepared while retaining the structure and function, thereby obliterating the difficulty in the membrane protein study in all biological areas including medicine and agriculture.

Because only water-soluble proteins containing ligands are separated by electrophoresis, any water-soluble protein can be analyzed free of various obstacles caused by contamination with water-insoluble protein.

Water-soluble proteins separated by one-dimensional or two-dimensional electrophoresis are directly blotted on a plate for mass spectrometry, thereby drastically shortening the operation time, enabling the detection of low-abundance proteins by increasing the amount of proteins to be transferred to the plate for mass spectrometry, downsizing and robotizing the device, and increasing the number of samples to be processed.

By bringing a liposome to which membrane protein(s) has been transferred into contact with various ligands immobilized on a support for purification utilizing their biological affinity, a highly purified objective membrane protein-ligand complex can be isolated.

According to the present invention, pure interaction between molecules of a membrane protein and a ligand (inclusive of competitive agent) can be measured, irrespective of whether the both molecules are highly purified or coexistent with a number of other substances, without an influence from an intracellular signal transmitter or a transcription regulator. In other words, an interaction detection method based on cell response leaves the physiological point of action of a ligand unidentified, whether it is a membrane protein, an intracellular signal transmitter a transcription regulator, or a different action point. In contrast, the present invention affords detection of interaction solely between a membrane protein and a ligand.
Purification of the membrane protein-ligand complex on a plate for mass spectrometry analysis enables collective detection of these two by MASS spectrometer.
The formation and detection of a membrane protein-ligand complex clarifies the function of the membrane protein (interaction with the ligand).
The present application also describes a method of identifying the membrane proteins and/or their ligands that show changes in the amount or properties specific in a certain disease, which method comprising analyzing proteome by the above-mentioned method using a sample taken from an organism (including human, plants, animals and all other organisms) suffering from the disease, and comparing the obtained analysis data with that of healthy homologous organism. When this method is applied to plural diseases and the obtained data are pooled, a database for diagnosis can be constructed. In this way, by finding disease-specific membrane proteins and their ligands, inputting the quantitation values into a database, and comparing the data with the data of healthy homologous organism, the diagnosis of diseases based on binary pharmacoptoteomics data of membrane proteins and ligands becomes available.

The pharmacogenomics has a ceiling imposed by genetic diathesis analysis of the patient or static etiology. The analysis results of pharmacogenomics are mostly not directly related to the disease of the patient, and therefore, cannot provide information of the proteins that change dynamically and cause the disease. In contrast, pharmacoproteomics directly teaches the dynamics of the protein controlling biological reactions and dynamically shows the current disease state of the patient through changes of the protein.

Collection of pharmacogenomic information conflicts the personal information relating to the genetic diathesis of the patient, which could be sometimes an irresolvable moral issues for individual, race and nation. However, since pharmacoproteomic information concerns clarification of the cause of the disease of the patient and is collected in the same way as in the test and diagnosis generally performed in hospitals, there is no concern of being involved in a struggle between the bioscientific view and the moral view of the 21st century.

Inasmuch as the binary data of membrane protein and ligand can be collectively analyzed, the cause of the disease can be diagnosed instantaneously when the disease is caused by the defective signal transduction via a membrane protein, if it is caused by changes in the amount and property of membrane protein, changes in the amount and property of ligand or both.

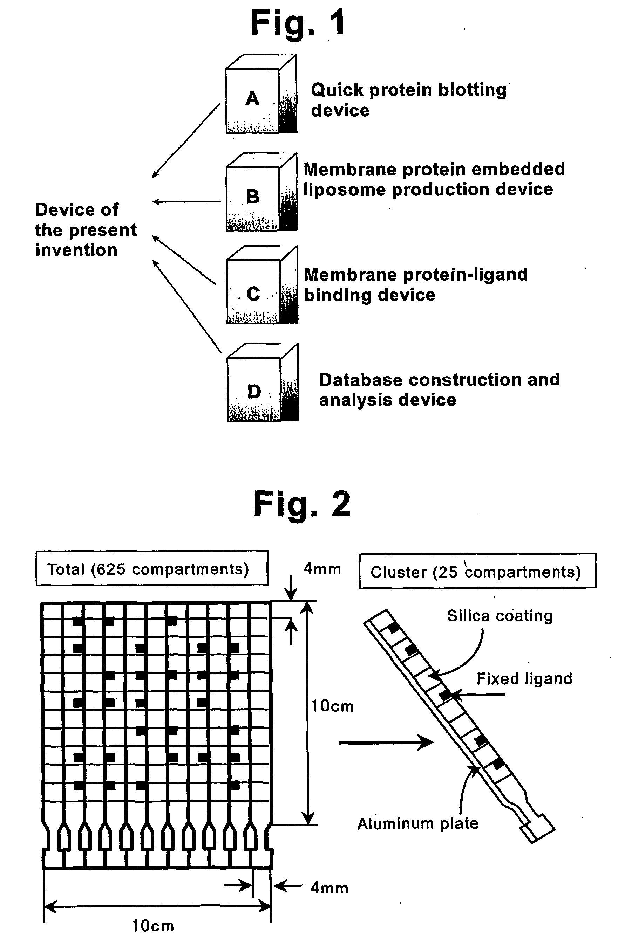

Once the aforementioned constitutions of the present invention are realized in a full automatic device, a revolutionary industrial field of utilization can be explored. On example thereof is a full automatic membrane protein-ligand proteome analysis system, whose elements are schematically shown in Fig. 1. It is needless to say that various other devices can be assembled based on the present invention according to the study object of membrane protein.

The present invention is applicable to finding, identification and analysis of all other types of receptors (GPI type, oligomer type) and their ligands that can be hardly found not only by research of orphan ligands involving predicting G protein coupled receptor (GPCR) from genomic sequence by homology search but also by prediction technology using computer and genome sequences based on homology.

Moreover, the present invention is considered to contribute enormously to finding, identification and analysis of all types of membrane proteins (membrane proteins other than receptors), that are still now extremely difficult, thereby enabling development of the so-called "membrane protein related drug discovery-type" medicines inclusive of the so-called "receptor related drug discovery-type" medicines, which aims at analysis of diseases, development of diagnostic agent and diagnostic method and development of therapeutic agent in which all membrane proteins and ligands are involved.

The present invention that introduces an epoch-making methodology into the study of membrane protein, which has been of utmost difficulty in the history of biological study, will not only contribute to the medical care but become a driving force of discovery and utilization of a new function of organisms as well as new industry of their applications, that aim at resolution of critical issues relating to the food and environment, which are the global problems of early in the 21^{st} century.

These and other objects and advantages of the present invention, and other characteristics of the present invention will become clear from the following description of the present invention.

### BRIEF DESCRIPTION OF THE DRAWINGS

Fig. 1 schematically shows a representative embodiment of the proteome analysis system and the elements thereof of the present invention.
Fig. 2 shows one embodiment of the plate for mass spectrometry used in the present invention.
Fig. 3 shows various embodiments of the water-soluble protein (ligand) support used in the present invention.
Fig. 4 shows one embodiment of the ligand-receptor (membrane protein) matrix table used in the present invention.
Fig. 5 shows expression of urokinase receptor (ligand: FITC-UK), C5a receptor (ligand: FITC-C5a) and interferon-γ receptor (ligand: FITC-INFγ) on a U937 cell membrane before (Fig. 5B) and after (Fig. 5A) Bt₂cAMP stimulation.
Fig. 6 is a photograph showing separation of a membrane fraction from U937 cell, wherein left shows before 40% sucrose density gradient centrifugation and right shows after 40% sucrose density gradient centrifugation.
Fig. 7 is a photograph showing isolation of U937 membrane protein embedded liposome, wherein A: liposome (200 µl) + membrane fraction; B: liposome (50 µl) + membrane fraction; C: membrane fraction; D: liposome (200 µl).
Fig. 8 shows the results of FACS analysis of FITC labeled membrane fraction (Fig. 8A), FITC labeled, membrane protein-embedded liposome (Fig. 8B) and simple liposome (Fig. 8C).
Fig. 9 is the result of Western blot analysis showing the expression of urokinase receptor on a U937 cell membrane.
Fig. 10 is a confocal laser photomicrograph of urokinase receptor embedded liposome in the presence (Fig. 10A) and in the absence (Fig. 10B) of anti-urokinase receptor antibody.
Fig. 11 is the result of Western blot analysis of a solubilized membrane protein-embedded liposome.
Fig. 12 shows a ligand (FITC labeled urokinase) binding ability of a urokinase receptor in a membrane fraction (Fig. 12A), wherein FITC labeled HSA was used as a control (Fig. 12B).
Fig. 13 shows a ligand (FITC labeled urokinase) binding ability of a urokinase receptor embedded in a liposome (Fig. 13A), wherein FITC labeled HSA was used as a control (Fig. 13B).
Fig. 14 shows molar ratio dependency of binding of a labeled ligand with U937 cell (Fig. 14A) and a urokinase receptor embedded liposome (Fig. 14B).
Fig. 15 shows a ligand (FITC labeled urokinase) binding ability of a membrane protein-embedded liposome prepared from PMA stimulated (Fig. 15B) and non-stimulated (Fig. 15A) U937 cell.
Fig. 16 shows appearance of a urokinase receptor embedded liposome by decreasing the liposome particle size.
Fig. 17 is a photograph of a plate for mass spectrometry on which urokinase is blotted and coomasie brilliant blue-stained polyacrylamide gels before and after blotting onto the plate.
Eg. 18 shows the results of mass spectrometric analysis of urokinase blotted on a plate for mass spectrometry.
Fig. 19 shows the results of quantitation of urokinase blotted on a plate for mass spectrometry.
Fig. 20 shows the results of mass spectrometric analysis of bacteriorhodopsin, wherein Fig. 20A shows the results at various concentrations of bacteriorhodopsin and Fig. 20B shows the results of bacteriorhodopsin alone, bacteriorhodopsin in the co-presence of a simple liposome and bacteriorhodopsin embedded in a liposome.
Fig. 21 shows the results of mass spectrometric analysis of a urokinase receptor on a plate for mass spectrometry, wherein Fig. 21A is photograph of electrophoresed purified urokinase receptor, Fig. 21B is a schematic drawing of a complex formed on a plate for mass spectrometry, and Fig. 21C shows the results of mass spectrometric analysis when anti-urokinase receptor IgG (upper) and control IgG (lower) were used as ligands.
Fig. 22 shows the results of mass spectrometric analysis of bovine rhodopsin with the addition of IAA or DHB conditions:
   bovine rhodopsin: 2.56 pmol membrane suspension (in 20 mM Tris-HCI, 10 mM β-mercaptoethanol, 100 µM EDTA, pH 7.5)
   matrix: 0.17 mg/µL DHB (in ethanol 0.5 µL) or IAA saturated solution (in ethanol 1.0 µL).
Fig. 23 shows selective determination of urokinase-receptor as a denatured protein model (Fig. 23A) and urokinase as a physiological protein model (Fig. 23B) by mass spectrometry.
Fig. 24 shows binding of a liposome with various receptors embedded and their ligands immobilized on Sepharose 4B gel.
Fig. 25 shows collective mass spectrometric analysis of urokinase as a ligand and urokinase-receptor as a receptor.

### DETAILED DESCRIPTION OF THE INVENTION

### 1. Terms and general embodiment of the present invention

The following terms when used in this specification generally mean the following. A general embodiment of the present invention is as follows.
(i) The term "complementary" means topological compatibility or conformity of the surfaces by which a receptor and a ligand thereof interact. In other words, a receptor and a ligand thereof are complementary, and therefore, the properties of the contact surface thereof are complementary with each other.
(ii) A "ligand" is a molecule recognized by a specific receptor. The ligand in the present invention is not limited to physiological one, and includes full agonist, partial agonist, antagonist and inverse agonist to a cell membrane receptor, toxin, viral epitope, hormone (e.g., sedative, opiate, steroid etc.), peptide, enzyme, substrate of enzyme, co-factor, drug, lectin, saccharide, oligonucleotide, nucleic acid, oligosaccharide, protein and monoclonal antibody. The ligand may be a natural molecule or an artificial molecule. The location of the natural ligand is not limited. It can be any substance present on the surface of the earth including aerosphere, substances secreted by organisms, intracellular substance, organelle substance, nuclear substance and others.
(iii) A "receptor" is a molecule having affinity for a specific ligand. The receptor may be a natural molecule or an artificial molecule. This functions alone or as a complex with other molecule species. The receptor forms a complex receptor (oligomeric receptor) by a covalent bond or a non-covalent bond directly or via a specific binding substance. The receptor used in the present invention includes, but not limited to, antibody, cell membrane receptor, monoclonal antibody and antiserum that react with a specific epitope (e.g., on virus, cell or other material), drug, polynucleotide, nucleic acid, peptide, co-factor, lectin, saccharide, polysaccharide, cell, cell membrane and organelle. The receptor is sometimes called "anti-ligand" in the pertinent field. When the term "receptor" is used in this specification, the difference in the meaning is not intended.

In the present invention, moreover, irrespective of its structure and function, any membrane receptor, membrane channel, membrane pump, membrane transporter, membrane lining protein and a substance incidentally binds with these proteins are broadly referred to as a receptor or a membrane protein. This is because those of ordinary skill in the art would easily recognize that the method of the present invention permits isolation and identification of them.

When two macromolecules bind via molecular recognition to form a complex, a "ligand-receptor complex" is formed. The location of a receptor is not limited to a cell membrane (so-called plasma membrane forming an outer layer of cell) in a narrow sense. A receptor refers to a molecule binding with any membrane having a common constituent lipid bilayer. For example, a DNA polymerase complex binding with a nuclear membrane is important to the replication and repair of DNA, and RNA polymerase is important to transcription, and a ribosome binding with an endoplasmic reticulum membrane is important to the translation of a protein. A group of oxidoreductases binding with a mitochondrial membrane plays an important role in ATP production, a group of metabolism associated enzymes of a peroxisomal membrane are involved in the metabolism of peroxide and generation of heat, a group of degrading enzymes contained in a lysosomal membrane are involved in the degradation of protein, nucleic acid, saccharide and lipid, and a membrane protein of Golgi apparatus has an important function in glycosylation after protein synthesis and membrane transport of synthesized protein or lipid. Furthermore, there is suggested a possibility of various intracellular membrane surfaces involved as a footing where a group of phosphorylated enzymes and dephosphorylated enzymes deeply involved in intracellular signal transduction act. The foregoing examples do not limit the important function of receptor (membrane protein) to the range exemplified above. The membrane proteins and their functions newly identified by the present invention, as the membrane protein-related life phenomena are shown to be diverse, are all encompassed in the present invention.

The positional relationship between a receptor and a lipid bilayer varies. Most common is of a transmembrane type (G protein-coupled receptor) folded several times and stabilized by hydrophobic interaction of a hydrophobic region of a protein and a hydrophobic region of a lipid bilayer. This includes a GPI anchor type receptor (glycosylphosphatidylinositol-anchored receptor) embedded in an outer lipid layer of a lipid bilayer. Examples include glycoprotein, glycolipid and oligomeric saccharide, which are immobilized on the outer surface layer of a cell, oligomeric receptor constituent molecule group in a wide sense inclusive of GTP/GDP coupled protein group which are immobilized inside a cell, membrane lining protein group playing an important role in the retention and changes in the shape of membrane, functional protein group bound therewith, and the like. The foregoing exemplifies the positional relationship between receptor (membrane protein) and lipid bilayer. The exemplification is not limitative, and the present invention encompasses any positional relationship involved as the present invention clarifies a wide variety of such relationships.

There are a number of receptors that can be the object of study in the present invention including unknown ones. The following exemplification recites only a part of them.

### a) cancer specific membrane protein

The development of a medicament having an action mechanism of growth suppression, apoptosis induction, metastasis suppression of cancer cells is expected by identifying a membrane protein that expresses and functions in a cancer cell membrane and analyzing its function. An antibody specific to this membrane protein itself can be used as an effective medicament and is also applicable to a targeting therapy for delivering toxin and the like to a target cancer cell.

b) By identifying a membrane protein that expresses in a cell membrane of a tissue attacked by an autoantibody (ligand) in an autoimmune disease or auto tissue-toxic lymphocyte in organ transplantation, related'diagnostic agents or medicines useful for blocking the bond with an autoantibody or autoantigen specific tissue-toxic lymphocyte can be developed. Particularly, diversity of disease in autoimmune diseases is considered to be based on tissue specificity, and if a membrane antigen that autoantibody or autotissue-toxic lymphocyte attacks in Addison's anemia, Glomerulonephritis (primary, IgA), Grave's hyperthyroidism, Insulin-dependent diabetes, Multiple sclerosis, Pernicious anemia, Rheumatoid arthritis, Sjogren's syndrome, Psoriasis, Systemic lupus erythematosus, Thyroiditis, Vitiligo, Crohn's disease, Idiopathic thrombocytopenic purpura, and other diseases could be isolated and identified, medicines with alleviated side effects, which is specific to each autoimmune disease, can be developed.

c) By specifying the endogenous ligand of benzodiazepine receptor, Cannabinol receptor, Sigama receptor 1 and Sigama receptor 2, with regard to which artificial ligands (competitive agent) are present but endogenous ligands are unknown, and by specifying the endogenous ligand binding with Phencyclidine binding site of NMDA receptor, an innovative therapeutic agent for central nerve diseases can be developed.

### d) Receptor expressed in microorganism

The determination of a ligand that binds to a membrane transporter essential for the survival of microorganisms is useful for the development of an antibiotic having a new action mechanism. Particularly, an antibiotic against opportunistic fungi, protozoa, and bacteria resistant to antibiotics now in use is valuable.

### e) Receptor for nucleic acid as ligand

When a nucleic acid sequence is synthesized and a membrane protein hybridizing to DNA or RNA sequence is isolated, identified, functionally analyzed, a completely new interaction between the exogenous nucleic acid and cell membrane function is elucidated, which in turn leads to the development of useful related diagnostic agents or medicines. For example, an effective transport system into the cell for medicines based on antisense technology or a new infection defense mechanism of DNA, RNA virus can be developed.

### f) Receptor for lipid or lipid metabolite as ligand

When these low molecular weight compounds are synthesized and a crossreacting membrane protein is isolated, identified, functionally analyzed, a useful related diagnostic agent or medicament can be developed. For example, medicines having completely new action mechanism in the areas of diseases of central nervous system, circulation system, cancer, diseases of digestive system or immune system can be developed by finding a completely new receptor involved in a smooth muscle contraction and relaxing action of many metabolites in the arachidonate cascade and a novel subtype of EDG (endothelial differentiation gene) receptor involved in morphology, relocation, growth and attachment of the cell.

### g) Membrane protein prion

For the elucidation of mechanism of conversion from normal prion to pathogenic prion, a reconstituted system wherein a GPI type prion membrane protein in its intact structure is embedded in a liposome is used, and a release mechanism from the liposome membrane, isolation of a release promoting molecule, analysis of conversion rate of membrane protein type prion and free prion into pathogenic prion and the like are studied based on the present invention. Consequently, a measurement system of pathogenic prion, a removal method of pathogenic prion, a pathogenic prion infection defense method, a CJD onset delaying method, CJD patient therapy method and the like can be developed.
(iv) The "biomembrane" refers to any membranes having a lipid bilayer as a component including cell membrane and membrane which constitutes organelle, such as, endoplasmic reticulum membrane, Golgi apparatus membrane, nuclear membrane and the like of any organism.
(v) The "liposome" means a particle partitioned from.the outside world by a lipid bilayer. The lipid bilayer of liposome is similar to biomembrane physically, chemically and biologically. Preferably the liposome is made of membrane component such as lipid extracted from a plant.
(vi) The "membrane associated substance" refers to any substance that penetrates or binds to inside or outside of a biomembrane. The membrane associated substance includes membrane receptor that transduct signals from outside to inside of a cell, membrane protein constructing membrane channel for transportation of physiological substance between outside and inside of a cell, membrane lining protein that retains kinetic membrane structure, protein translation enzyme, ribosome and any other substance binding to biomembrane via covalent or non-covalent bond.
(vii) The "membrane protein embedded liposome" refers to a liposome on which membrane proteins bind via covalent or non-covalent bond.
(viii) The "membrane protein library" means a set of membrane protein embedded liposomes made of membrane proteins contained by a biomembrane of a certain cell, all biomembrane of a certain cell, all biomembrane of a certain tissue, all biomembrane of a certain organ, all biomembrane of a certain individual or any other possible biomembrane sample.
(ix) By the "ligand support" is meant a substrate to which soluble molecules generally called ligands binds. This substrate consists of a basic structure to maintain its shape and properties and a ligand adsorbing material (surface) to optimize the binding mode and binding amount of the ligand.
   A ligand adsorbing material used in connection with the methods of the present invention may be a covalent bonding or non-covalent bonding adsorbing material depending on the binding mode of the ligand. The latter adsorption mode typically includes normal phase, reverse phase, hydrophobic, anionic, cationic and other non-covalent bonding adsorbing materials.
   An adsorbing material used in connection with the methods of the present invention may contain a spacer molecule to take the distance (10 Å - 10000 Å, preferably about 100 Å) between the ligand and the surface of the basic structure of the substrate. The material of this spacer molecule may be a net or porous biological polymer or synthetic polymer. In any case, it is formed to afford avidity rather than affinity of membrane protein and ligand to allow binding of a membrane protein embedded in a liposome having a diameter of 10 nm - 5000 nm with a ligand with a large binding force.
   In most embodiments, the shape of the substrate is substantially planar but in some embodiments, it is in the form of magnetic particle, non-magnetic particle, strand, precipitate, gel, sheet, tube, spherical body, container, capillary, pad, slice, film, plate, slide and other various surface structures. Essentially, an optional convenient form can be employed in the present invention.

The surface of the substrate may be biological, non-biological, organic or inorganic, or a combination of these, and is formed on the surface of a hard support on which the reaction described in the present specification occurs.

The surface of the substrate is selected to afford suitable protein adsorbing property. Examples thereof include functionalized glass, Si, Ge, GaAs, GaP, SiO₂, SiN₄, reforming silicone, a wide range of gels and polymers, such as (poly)tetrafluoroethylene, (poly)vinylidene difluoride, polystyrene, polycarbonate, or a combination of these and the like. Examples of the surface of the substrate having plural monomer or polymer sequences include linear or circular polymers of nucleic acid, polysaccharide, lipid, peptide having α-, β- or ω-amino acid; gel surface carrier used for chromatography (anionic/cationic compounds, hydrophobic compound consisting of 1 to 18 carbon chains, carrier crosslinked with hydrophilic compound, such as silica, nitrocellulose, cellulose acetate, agarose and the like, etc.); synthetic homopolymers such as polyurethane, polyester, polycarbonate, polyurea, polyamide, polyethyleneimine, polyallylenesulfide, polysiloxane, polyimide, polyacetate and the like; heteropolymers that are conjugates of any of the above-mentioned compound and a known drug or natural compound bound thereto (covalent or non-covalent bond); and other polymers that will be found suited after general view of this disclosure.
(vii) By the "plate for mass spectrometry" is meant, of the substrate (ligand support) for binding a soluble molecule generally referred to as a ligand, a substrate capable of automatically and instantaneously adapted after separation of ligands by a high precision analysis method, such as one-dimensional and two-dimensional electrophoresis, high performance liquid chromatography and the like and direct transfer onto the substrate surface, to the subsequent highly sensitive mass spectrometer. The substrate consists of a basic structure to maintain its shape and properties and a ligand adsorbing material (substrate surface) to optimize the binding mode and binding amount of the ligand.
   A ligand adsorbing material described herein may be a covalent bonding or non-covalent bonding adsorbing material depending on the binding mode of the ligand. The latter adsorption mode typically includes normal phase, reverse phase, hydrophobic, anionic, cationic and other 0-covalent bonding adsorbing materials.
   An adsorbing material described herein may contain a spacer molecule to take the distance (10 Å - 10000 Å, preferably about 100 Å) between the ligand and the surface of the basic structure of the substrate. The material of this spacer molecule may be a net or porous biological polymer or synthetic polymer. In any case, it is formed to afford avidity rather than affinity of membrane protein and ligand to allow binding of a membrane protein embedded in a liposome having a diameter of 10 nm - 5000 nm with a ligand with a large binding force.

The surface of the substrate may be biological, non-biological, organic or inorganic, or a combination of these, and is formed on the surface of a hard support on which the reaction described in the present specification occurs.

The surface of the substrate is selected to afford suitable protein adsorbing property. Examples thereof include functionalized glass, Si, Ge, GaAs, GaP, SiO₂, SiN₄, reforming silicone, a wide range of gels and polymers, such as (poly)tetrafluoroethylene, (poly)vinylidene difluoride, polystyrene, polycarbonate, or a combination of these and the like. Examples of the surface of the substrate having plural monomer or polymer sequences include linear or circular polymers of nucleic acid, polysaccharide, lipid, peptide having α-, β- or ω-amino acid; gel surface carrier used for chromatography (anionic/cationic compounds, hydrophobic compound consisting of 1 to 18 carbon chains, carrier crosslinked with hydrophilic compound, such as silica, nitrocellulose, cellulose acetate, agarose and the like, etc.); synthetic homopolymers such as polyurethane, polyester, polycarbonate, polyurea, polyamide, polyethyleneimine, polyallylenesulfide, polysiloxane, polyimide, polyacetate and the like; heteropolymers that are conjugates of any of the above-mentioned compound and a known drug or natural compound bound thereto (covalent or non-covalent bond); and other polymers that will be found suited after general view of this disclosure.
(viii) By the "mass spectrometer" is meant a device that measures and detects molecular weight of a substance, by ionizing a sample in a gaseous state, casting the ionized molecules and molecule fragments thereof into an electromagnetic field, separating them according to the mass number/charge number based on the migration state, and determining the spectrum of the substance. There are several types that can be preferably utilized, but other types can be also used.

In a typical embodiment (Fig. 1), the proteome analysis method of the present invention comprises a step for separating only water-soluble proteins by gel electrophoresis and blotting the separated water-soluble proteins directly onto a plate for mass spectrometry from the gel (step A), a step for attaching or penetrating the membrane protein to or into an artificial liposome membrane (step B), a step for bringing a membrane protein embedded liposome into contact with a plate for mass spectrometry on which a water-soluble protein is blotted and forming a ligand-receptor complex utilizing biological affinity (avidity) (step C), and a step for collectively detecting the complex by mass spectrometry and pooling and making a database of the obtained data (step D).

The specific embodiments of the steps A to D and each device for practicing the steps and other aspects of the present invention derived from the constituent elements are explained in detail in the following.

### 2. Quick protein blotting device (device A)

This device consists of an electrophoretic device, a protein blotting device and a plate for mass spectrometry as main constituent elements. The electrophoretic device may be commercially available or devised specially. According to the object, both the one-dimensional gel electrophoresis and two-dimensional gel electrophoresis can be used. In the two-dimensional gel electrophoresis, the first migration is based on the separation by the isoelectric point of the protein, and the second migration is based on the separation according to the molecular weight of the protein. The size of the gel used for electrophoresis is not subject to any particular limitation. While 10 cm × 10 cm is typical, but 20 cm × 20 cm or other sizes can be used where necessary. While the basic material of the gel is polyacrylamide, a different substrate such as agarose gel, cellulose acetate membrane and the like can be also used depending on the purpose. The gel concentration may be constant or may have a gradient.

The water-soluble protein to be subjected to electrophoresis can be prepared from a biological sample by any known method. Examples of the sample include, but not limited to, cell, tissue or extracellular fluid (e.g., blood, plasma, urine, bone marrow fluid, ascites etc.) of optional organism such as plant, animal, microorganism and the like. For example, a soluble fraction can be obtained by, after obtaining the target cell, homogenizing in a suitable buffer solution in the presence of various protease inhibitors, or suspending with a cell homogenizer such as Polytron and the like, or rupturing the cell by hypoosmotic shock, or rupturing the cell membrane by ultrasonication, and then centrifuging to obtain a supernatant.

The second step includes transferring the protein from the gel after electrophoresis onto a plate for mass spectrometry. In a preferable embodiment of the present invention, the shape of the plate for mass spectrometry is devised to completely match the sample inlet of the mass spectrometer to be used thereafter. For example, a "clustered plate for mass spectrometry" is mentioned, which has split lines previously made to allow easy separation of the cluster into each split type plate, which fits the sample inlet of the mass spectrometer, after transfer of proteins to a cluster type plate for mass spectrometry fitting the size of a gel after electrophoresis. Fig. 2 shows one embodiment of a preferable plate for mass spectrometry of the present invention.

In conventional mass spectrometer, samples are measured one by one, or only limited samples are measured at a time, because of one dimensional transfer of the plate for mass spectrometry, like the aforementioned split type plate for mass spectrometry. In contrast, the completely automated proteomics analysis according to the present invention enables analysis of all the proteins spread two-dimensionally by electrophoresis by moving the laser nozzle or a table carrying the plate for mass spectrometry in the two-dimensional directions for continuous whole scanning (intermittent scanning leaves proteins free of laser beam irradiation, namely unanalyzed proteins). This method combined with intermittent scanning makes it possible to mount 96 kinds of samples dispensed to a 96-well plate altogether on a plate for mass spectrometry (96 kinds of samples are arranged at the same intervals on a rectangular chip) and directly apply the chip to mass spectrometer.

In a preferable embodiment, the material of the plate for mass spectrometry consists of aluminum plate as a base and silica as an adsorbent material. The aluminum plate is used for blotting by an electric force, and a different conductor, like stainless steel, is also usable. For blotting by diffusion, an insulator (ceramic, plastic and the like) can be used. As the adsorbent material, silica on which any protein can be transferred is used, but other materials mentioned above can be also used depending on the object.

The protein spread in the gel after migration is transferred onto a plate for mass spectrometry by various methods (diffusion, electric force etc.). This step is generally called blotting. The efficiency of blotting is an important factor in relation to the measurement limit of the subsequent mass spectrometric analysis. In addition, denaturing of protein during migration and blotting should be avoided as far as possible. This is because when a water-soluble protein binds to a membrane protein, if one of them is denatured or the native three-dimensional structure is not maintained, the binding will not be completed. For this device to be applicable broadly to the study of proteomics, the composition of the molecules that cover the surface of the plate for mass spectrometry is significant. To improve efficiency of blotting, greater adsorption capacity of protein, capability of adsorbing any protein (rather than adsorption ability for specific protein) and constant adsorption rate for any protein are required, and to realize binding with a membrane protein, retention of the three-dimensional structure of protein, after blotting and adsorption onto a plate for mass spectrometry, are essentially required. Depending on the material of the surface, the membrane protein and ligand collectively, or membrane protein alone, or a ligand alone may be detected, identified and quantitated by MASS analysis.

When a membrane protein is analyzed by MASS spectrometry in the present invention, spacer made from a protein or other biopolymer or a synthetic polymer is preferably interposed between a ligand and a support, thereby to achieve efficient binding of the membrane protein and the ligand based on their biological affinity (avidity), because the membrane protein is immobilized on a liposome and the ligand, too, is immobilized on a support. As stated under "Terms and general embodiment of the present invention", the avidity needs to be improved. The biopolymers used in Examples are Protein G, an antibody and biotin. The distance between the ligand and the surface of the basic structure of the substrate is 10 Å - 10000 Å, preferably about 100 Å. The shape of the spacer may be a net or porous.

The binding mode between the support and spacer, and between a spacer and ligand is stated in detail later, but either a covalent bond or a non-covalent bond can be employed depending on the object of the proteome analysis. In accordance therewith, both the membrane protein and water-soluble protein can be collectively or respectively measured highly sensitively by mass spectrometry.

According to the method of the present invention, a protein migrated in a gel is blotted on a plate for mass spectrometry by a single step of treatment. Therefore, all the complicated steps after electrophoresis and before start of the mass spectrometric analysis needed in conventional proteomic analysis can be omitted, drastically shortening the operation time. Consequently, processing of an enormous amount of samples, which is necessary for proteome analysis, can be available. Moreover, in the present invention, a step for cutting the gel after electrophoresis into pieces is not necessary. As a result, proteins on the cut point (line) and loss fractions free of MASS determination are obliterated. This makes it possible to add all proteins in the gel to a plate for mass spectrometry. Instead of the step for extracting protein from the gel (extraction efficiency less than 10%), moreover, direct blotting by an electric force or diffusing power is employed, which makes 100% blotting efficiency realizable, and the amount of the protein blotted on a plate for mass spectrometry is several to several dozen times as much as the conventional amount of extracted protein.

In a typical embodiment of the present invention, a membrane protein and a ligand are collectively detected and analyzed, after forming a complex of a membrane protein-embedded liposome and a ligand as mentioned below, and in a different embodiment, the present invention provides an analysis method of a ligand by subjecting only a plate for mass spectrometry, on which the ligand (water-soluble protein) is blotted, to mass spectrometry.

That is, the protein on a plate for mass spectrometry can be analyzed by subjecting the plate for mass spectrometry as is to mass spectrometry. The analysis of the ligand fixed on a plate for mass spectrometry according to the present invention can be performed using any type of commercially available mass spectrometer, but more preferably done using a mass spectrometer that utilizes; the MALDI-TOFMS method consisting of matrix-assisted laser desorption/ionization (MALDI) wherein a sample is mixed with a matrix that absorbs laser beam and dried to allow crystallization, the crystallized sample is then ionized and introduced into vacuum by energy transfer from the matrix and by high-intensity laser pulse, and of time-of-flight mass spectrometry (TOFMS) wherein the mass number is analyzed based on difference in the times of flight of sample molecule ions caused by initial stage acceleration; a method wherein one protein is placed in one liquid drop and is ionized directly and electrically from the liquid; a nano-electrospray mass spectrometry (nano-ESMS) method wherein sample solution is electrically sprayed into the air, and each unfolded protein multivalent ion is led into a gaseous phase, and the like.

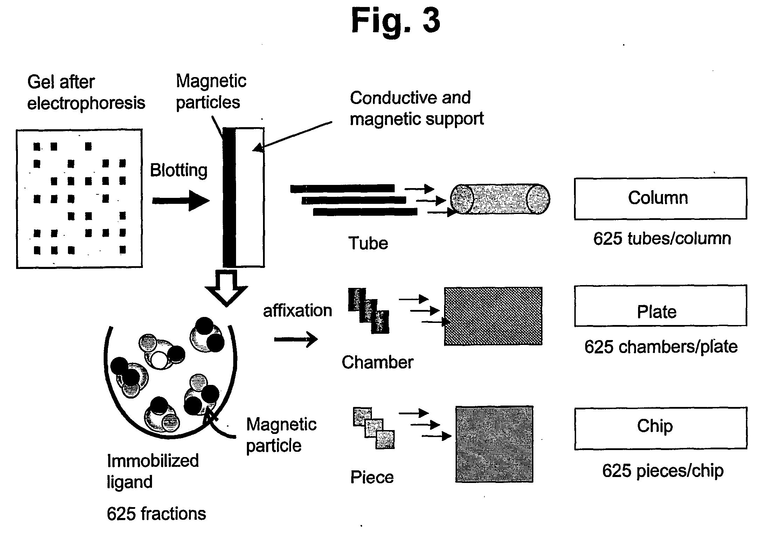

Various options are available for the ligand support of the present invention by combining a support most suitable for the target and object of proteome analysis and ultra-highly sensitive detection system. The detail is given below (for example, Table 1 and Fig. 3), and non-magnetic particles and magnetic particles are exemplified besides the plate for mass spectrometry.

Various options are available also for the detection system of the present invention in combination with a ligand support most suitable for the target and object of the proteome analysis. The detail is given below and fluorescence determination, radioactivity determination and the like are exemplified besides the mass spectrometry. When the ligand support is particles, the membrane protein-ligand complex can be highly sensitively detected, isolated and quantitated by the use of a liposome fluorescent-labeled in advance. This step (step A) can be applied to not only water-soluble protein but also to peptide, saccharide, DNA, RNA etc. present in extracellular fluid (e.g., various body fluids such as blood, plasma, urine, bone marrow fluid, ascites etc.), intracellular fluid or intra-organellar fluid, without attaching to or penetrating a cell-constituting lipid bilayer such as cell membrane, nuclear membrane, endoplasmic reticulum membrane and the like, any other soluble molecule derived from the body, any artificially synthesized compound, gaseous substance (e.g., oxygen molecule, nitrogen oxide etc.) and the like. That is, utilizing the properties, each analysis target substance is highly purified, transferred to an optimal support and subjected to an analysis of interaction with a liposome encapsulating a membrane protein and the like.

### 3. Device to produce Membrane protein-embedded liposome (device B)

This device includes devices for separation of membrane fraction from a cell, preparation of liposome, fusion of membrane fraction and liposome, adjustment of particle size of fusion liposome (membrane-protein-embedded liposome), and where necessary, preservation of fusion liposome.

For extraction of a membrane fraction from a cell, conventional methods can be used. For example, a target cell is obtained and homogenized in a suitable buffer solution in the presence of various protease inhibitors, or suspended in a cell disruption device such as Polytron and the like, or ruptured by a low osmotic pressure shock, or a cell membrane is destroyed by ultrasonication. Thereafter, the cell membrane fractions and organelle membrane fractions are prepared by density gradient centrifugation using various media.

As a method for the preparation of liposome, various known methods can be used. Typically but not limited to, a mixture of selected lipid is homogeneously dissolved in an organic solvent, the solvent is completely vaporized in argon gas and hydrated in a buffer solution to generate a liposome. The composition of liposome is important. In general, a cell membrane contains cholesterol abundantly, but lipid bilayer constituting organelle such as endoplasmic reticulum and the like contains a little or no cholesterol. Therefore, the composition of the liposome to receive membrane protein becomes an extremely critical factor in determining which membrane protein of the cell is to be transferred to the liposome. Those of ordinary skill in the art can determine appropriate lipid constituting the liposome depending on the derivation of the membrane protein.

As a method for fusion of membrane fraction and liposome, various known methods can be used. For example, a method including mixing the both in a suitable proportion and then repeating freezing-thawing, a method including placing an aqueous solution containing a membrane fraction on a film made from a liquid mixture of a selected lipid and then transferring the membrane protein to the lipid bilayer by hydration, or a different method can be used for this purpose. Preferably, it is a freeze-thaw method, and the use of this method enables achieving a 100% embedding ratio (transfer ratio) of membrane protein to liposome.

Alternatively, when the fusion with a membrane protein is realized after a liplsome is formed, the fusion of liposome and membrane protein fraction can be promoted by adjusting the particle size to 10 nm - 5000 nm, preferably 10 nm - 500 nm.

The prepared membrane protein-embedded artificial liposome can be sized by ultrasoniation, homogenizing or other method. In the present invention, it is preferable to use filtration (extruder method) to minimize denaturing of the membrane protein and to adjust the particle size 10 nm - 5000 nm, preferable 10 nm - 500nm.

By carefully adjusting the mixing ratio of the membrane fraction and liposome, the desired kind and number of membrane proteins to be embedded to a liposome can be controlled. This technique is absolutely important to facilitate the analysis by reducing the noise proteins (noise peaks) in measuring and determining the molecular weights of both the membrane protein and ligand that formed the complex of the present invention by device C to be mentioned below.

The development of a method for stably preserving a liposome thus obtained comprising the target membrane protein attached to or penetrating a lipid bilayer (membrane-protein-embedded liposome) is extremely important to make the study of proteomics available anywhere any time and at any institutions that do not have mass spectrometer and the like. Several additives developed for preservation of a simple liposome can be used for this end.

The method of the present invention can be applied irrespective of the kind of the membrane protein. Therefore, it is applicable to finding, identification and analysis of all other types of receptors (GPI type, oligomer type) and their ligands that can be hardly found by genomic sequence homology based prediction with computer as well as to searching the orphan ligand of G protein coupled receptor (GPCR) predicted from genomic sequence by homology search, whereby the development of medicines by receptor based drug discovery targeting all types of receptors becomes possible. In addition, the present invention contributes enormously to finding, identification and analysis of membrane proteins other than the receptor. Therefore, the development of medicines by membrane protein based drug discovery aiming at the analysis of the diseases involving all membrane proteins and ligands, the development of diagnostic agents and diagnostic methods, and the development of therapeutic agents becomes attainable.

The method of the present invention can be applied to finding, identification and analysis of intracellular membrane protein that normally does not appear on the surface layer of cell membrane, such as one immobilized (penetrated) on the inside layer of a lipid bilayer by its constituent lipid moiety, and one immobilized on the inside layer of a lipid bilayer generally called lining protein of biomembrane.

Furthermore, the present invention contributes enormously to the study of antibody based drug discovery (inclusive of antibody drugs) aiming at using an antibody for the analysis, diagnosis and therapy of diseases. Antigens effective for the analysis, diagnosis and therapy of diseases are present as membrane proteins, and for the preparation of the corresponding antibodies, a membrane antigen (membrane protein) retaining structure and function is indispensable. Only the method of the present invention, wherein a membrane antigen is embedded in a liposome while retaining structure and function, can satisfy the above-mentioned condition essential for the antibody based drug discovery. It is evident that the antibody related drug discovery method is one special method substituting a ligand with an antibody in the general methods aiming at the development of analysis, diagnostic agent and diagnosis method of diseases involving all membrane proteins and ligands, and development of therapeutic agents, which the present invention covers. Therefore, the present invention affords development of antibody drug discovery type medicines in addition to the development of receptor drug discovery type medicines.
The present invention enables, for the proteomic analysis of the membrane protein and its ligand, collective finding and collective quantification, as well as functional (interaction) analysis of both membrane protein and ligand thereof, or finding and quantitation of either one and analysis of the interaction of the both. In this case, whether the membrane protein and its ligand are known or otherwise is not questioned. The ligand includes inherent endogeneous ligand, competitive agent (full agonist, partial agonist, antagonist, inverse agonist), medicine, reagent, antibody and any other substance artificially modified to bind with the membrane protein.
The membrane protein-embedded artificial liposome obtained in this step (step B) are suitable to be administered directly to a patient with a disease caused by defect, mutation and other abnormality of the membrane protein, by giving a normal membrane protein as a new dosage form membrane protein-embedded artificial liposome medicine. When the constituent components of the lipid bilayer are obtained from a biological species other than animal, human infection with a pathogen can be avoided.

A complex of a membrane protein-embedded artificial liposome obtained in this step and an endogeneous ligand, or a complex of a membrane protein-embedded artificial liposome and a competitive agent (irrespective of full agonist, partial agonist, antagonist or inverse agonist) are suitable to be administered directly to a patient with a disease caused by an abnormality of both or either of the membrane protein and the ligand, as a new dosage form membrane protein-embedded artificial liposome medicament having a new action mechanism.

The present invention can also provide the principle, methods and devices of detection of novel and ultrahighly sensitive membrane protein - ligand (inclusive of competitive agents) interaction by using, singly or in combination, a method of crosslinking a fluorescent substance and other label substance to a lipid bilayer of an artificial liposome by a covalent bond or non-covalent bond, a method of inserting by hydrophobic interaction and a method of encapsulating a soluble label substance in an aqueous phase in an artificial liposome. It is also possible to crosslink, insert or encapsulate a substance generating a secondary signal (e.g., enzyme such as alkaline phosphatase), instead of a fluorescent substance and other label substances, with or in an artificial liposome by utilizing an avidin-biotin system, nickel-histidine or other crosslinking system and amplify the detection sensitivity.

As shown in the Examples below, when an artificial liposome with labeled membrane protein(S) embedded, wherein fluorescent molecules and other molecules for labeling recognizable by FACS are introduced at a DNA level, RNA level or protein level by genetic engineering or other imaginable method, is sized to 10 nm - 5000 nm, preferably 500 nm or below, by an extruder method or other methods, FACS analysis clearly shows population of the labeled membrane protein-embedded liposome in the corresponding size area. Therefore, for example, when a cDNA coding a membrane protein is prepared from a gene sequence, and a host cell is transformed with an expression vector containing said cDNA, and a membrane protein (into which fluorescent molecule or other molecule for label

recognizable by FACS are introduced) is expressed, a labeled membrane protein-embedded non-labeled liposome prepared from a cell membrane fraction of the expression host is adjusted to said size and analyzed by FACS to detect the presence or otherwise of the expression of the membrane protein, and the expression amount thereof. Unlike other methods currently available, detection under the conditions without information other than the DNA sequence of the membrane protein or reagents (antibody, ligand, competitive agent, cell response or other detection agent of the membrane protein) becomes possible. With the presence of a detection agent of the membrane protein such as antibody and the like, labeling at a protein level becomes possible, and likewise, analysis of expression by FACS becomes possible. It is needless to say that a similar principle is applicable to the investigation of ligand.

This step is applicable to attaching or penetrating, besides the membrane protein, one or plural biological polymer(s) including saccharide, DNA, RNA that attach to or penetrate a cell constituting lipid bilayer, such as cell membrane, nuclear membrane, endoplasmic membrane and the like, to a lipid bilayer of an artificial liposome having predetermined lipid composition and shape.

### 4. Device for binding of membrane protein and water-soluble protein (device C)

This device includes a device for binding reaction of ligands blotted on a plate for mass spectrometry and membrane protein-embedded liposomes, a device for washing and removing a liposome non-specifically bound on a plate for mass spectrometry and a device for dissolving and removing the liposome to form complexes of only ligands and membrane proteins on a plate for mass spectrometry. For example, these devices may be a reaction vessel having a bottom area sufficient to immerse a plate for mass spectrometry in a reaction mixture, a washing solution and the like, which is equipped with a shaking means where necessary. It is possible to perform these plural steps in a single device, or a certain device may be omitted depending on the object.

In a binding reaction device, a step for coating (blocking) the adsorptive surface of a plate for mass spectrometry with a suitable substance, for the purpose of preventing a non-specific adsorption reaction on the plate for mass spectrometry other than physiological ligand-membrane protein interaction, can be applied as a pretreatment of the binding reaction. The requirements for a blocking agent are that it can prevent non-specific adsorption of hydrophilic head of the lipid bilayer, it can prevent non-specific adsorption of membrane protein, other than the target membrane protein, which has been transferred to the liposome, it is not a multicomponent system that makes subsequent mass spectrometric analysis unattainable but a system wherein its components are known and uniform in molecular weight, it consists of molecules that do not absorb ionization energy, and the like.

As the reaction method, simple immersion, shaking and the like may be employed. As a method for increasing the concentration of a membrane-protein-embedded liposome near the plate for mass spectrometry, concentration of liposome by electric force is recommended. Inasmuch as a liposome is negatively charged as a whole, liposome transfers to the surface of a plate for mass spectrometry and concentration near the ligand increases, once cathode is set at the bottom of the plate for mass spectrometry and anode at the upper part of the reaction tank.

The liposome non-specifically bound onto the plate for mass spectrometry can be removed by washing with a washing buffer having an appropriate salt concentration and composition. While the temperature conditions during washing are important, those of ordinary skill in the art can determine suitable conditions if necessary.

The method for removing the liposome by lysis is exemplified by a method comprising bringing a suitable organic solvent (glycerol, acetonitrile, alcohol, dioxane, DMSO, DMF and the like) in contact with a plate for mass spectrometry after adjusting its concentration as appropriate with a buffer. The use of a mild detergent (e.g., octylglucose and the like) is also effective.

The foregoing is the specification and explanation of the full automatic membrane protein-ligand proteome analysis device shown in Fig. 1, assuming mass spectrometric analysis as the detection system. In consideration of the compatibility with various objects of the present invention, the embodiments of the support of the water-soluble protein should be naturally diversified as shown in Fig. 3 and Table 1, which go beyond the range exemplified in Fig. 3 and Table 1.

**Table 1 Type and use of water-soluble protein (ligand) support**

| | Ligand | | Target | | |
|---|---|---|---|---|---|
| Type | Support | Binding manner | Ligand | Receptor | Application range |
| Column | Magnetic particle | Covalent | | O | LC-MS-MS [Entirely automated] |
| Plate | Magnetic particle | Covalent | O | | HTS (competitive agent screening) |
| Plate | (Direct) | Covalent | O | | HTS (competitive agent screening) |
| Chip | Magnetic particle | Covalent/ Non-covalent | O | O | Detection/identification of ligand (non-covalent) |
| Chip | (Direct) | Covalent/ Non-covalent | O | O | Detection by SELDI, plasmon, fluorescence, RI, etc. |
| | Non-magnetic particle | Covalent | O | O | Detection/discovery by fluorescence, RI, etc. |

In the example shown in Fig. 3, a conductive magnetic metal was used as a basic structure of the support to be blotted after electrophoresis, and magnetic particles bound with a spacer aiming at improved avidity and developed by the present invention were used as a surface material of the support. The magnetic particles were bound with water-soluble proteins separated by two-dimensional electrophoresis by a covalent or non-covalent bond, and in the example using a COLUMN, after division into 625 compartments, retained in the respective 625 microfiber lumens with a magnetic force. A membrane protein-embedded liposome was passed and passage, washing and eluted fraction were sequentially and respectively measured by LC-MS-MS. In a different application, using a PLATE, magnetic particles are retained in the respective 625 chambers with a magnetic force after divided into 625 compartments. Addition of a membrane protein-embedded liposome and a competitive agent, reaction, washing and detection were achieved at an HTS mode. CHIP application is an example applicable to the production of the plate for mass spectrometry exemplified earlier. In Table 1, one embodiment in the applicable range of the present invention depending on the kind of the support, wherein the lastly exemplified shows binding of water-soluble protein with a membrane protein-embedded liposome after covalently binding water-soluble protein to non-magnetic particles (polysaccharide gel, synthetic polymer gel and the like) via the spacer molecule of the present invention. As the detection system, fluorescence, radioactivity, secondary signal amplification system etc. other than mass spectrometric system can be flexibly employed according to the object of the proteomic analysis, thereby providing an optimal system for the search of unknown ligand, particularly orphan ligand and the like.

### 5. device for mass spectrometric analysis

The detection of the physiologically active substance used in the present invention is not limited to mass spectrometer. Nevertheless, mass spectrometer is considered an important detection device in the present invention, because a molecular weight, which is one of the physical amounts inherent to substance, can be directly measured, the detection limit is near picogram, and amino acid sequence can be analyzed by an MS-MS method. The analysis of a ligand-membrane protein complex immobilized on a plate for mass spectrometry of the present invention by the above-mentioned device C is adoptable to any type of commercially available mass spectrometers. For example, a mass spectrometer preferably used for the analysis of only a ligand immobilized on a plate for mass spectrometry by the above-mentioned device A can be used similarly.

In the present invention, when membrane protein and water-soluble protein are co-present on a plate for mass spectrometry, the both at the same time or only either one can be measured highly sensitively by mass analysis depending on the selection of the solvent to be added to the sample.

### 6. Device for Database construction and analysis (device D)

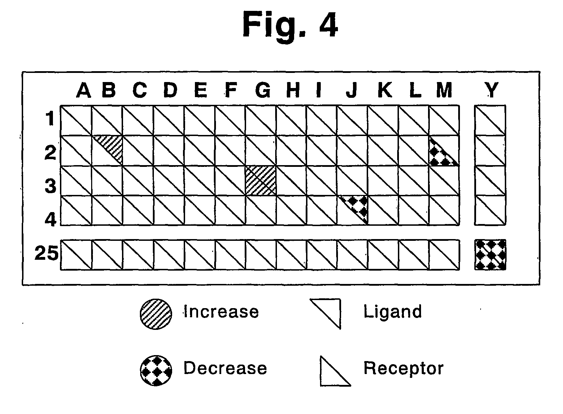

The measurement results of the above-mentioned membrane protein-ligand complex, which are obtained from the above-mentioned devices A, B and C of the present invention, are input to a "ligand-receptor (membrane protein) matrix table" (Fig. 4) previously set and new data are added at any time to construct a database for diagnostic determination.

Line numbers (1-25) and row symbols (A-Y) are assigned to cover the entire area of the plate for mass spectrometry introduced exemplarily in the above, thereby to allocate numbers (A1-Y25) to each compartment (4 mm × 4 mm) of total 625 (25 × 25) compartments in one-to-one correspondence (Fig. 2). As a result, the entire ligand transferred onto the plate for mass spectrometry after electrophoresis can be sorted out by the ligand-receptor (membrane protein) matrix compartment numbers. Needless to say, the corresponding receptor obtained thereafter by reaction with a receptor-embedded liposome can be sorted out by the same compartment numbers, and a substance group that gathers under a certain compartment number is presumed to bind mutually and physiologically.

In one specific embodiment, a target body fluid (considered to contain a soluble ligand) such as serum and the like of a healthy subject and the same kind of target body fluid of a patient having a specific disease are separately applied first to two-dimensional gel electrophoresis and, after blotting to a plate for mass spectrometry, applied to mass spectrometry and the like for the measurement. At this point, increase and decrease of ligand due to the disease becomes clear and can be entered in the database.

Then, a target body fluid, such as serum and the like, of a healthy subject is electrophoresed and transferred on two sheets of gel to prepare two plates for mass spectrometry, on which the ligand of the healthy subject has been transferred, are prepared. Separately, the same kind of cells are recovered from the healthy subject and the patient, and respective membrane proteins are transferred to liposomes using the device B device for membrane protein separation and transfer to liposome) mentioned above to prepare membrane-protein-embedded liposomes. The membrane-protein-embedded liposomes derived from the healthy subject and the patient are individually contacted with respective plates for mass spectrometry, on which the ligand of the healthy subject has been transferred using the device C (device for binding water-soluble protein and membrane protein), and a ligand-receptor binding reaction is carried out, which is followed by washing and the like, to obtain a ligand-receptor complex highly purified on the plates for mass spectrometry. By the mass spectrometric analysis, increase and decrease of receptor due to the disease becomes clear and can be entered into the database.

The items of the database are name of disease, name of body fluid from which ligand is obtained, name of cell from which membrane protein is obtained, as well as ligand-receptor (membrane protein) matrix compartment number, molecular weight or quantification value (or signal intensity output from analysis device such as peak height, peak area and the like) of the results of mass spectrometry, and the like.

The results of mass spectrometric analyses are input to the database according to an automating program and the results thereof are expressed in a differential display as shown in Fig. 4 wherein, in the triangle on the right half of "ligand/receptor (membrane protein) matrix table", an increase of ligand is shown with red color, a decrease with blue color and absence of change with yellow color and, likewise in the triangle on the left half, an increase of receptor is shown with red color, a decrease with blue color and absence of change with yellow color. In Fig. 4, patterns are employed.

Approximate manipulation time of the above is; overnight (about 12 hours) for simultaneous electrophoresis of 4 sheets, about 6 hours for simultaneous preparation of 2 kinds of membrane fractions, about 2 hours for simultaneous translocation to liposomes of 2 kinds of samples, about 1 hour for simultaneous ligand-receptor binding reaction of 2 kinds (total time for the above steps about 21 hours), about 52 hours of analysis of 625 compartments by commercially available TOF-mass spectrometer. When one mass spectrometer is used, total steps 21 + 52 × 4 = 229 hours are necessary, and when 4 mass spectrometers are used, 21 + 52 × 1 = 73 hours are necessary. The rate-determining steps are electrophoresis, preparation of membrane fraction and mass spectrometric analysis. When these are improved and automated, the manipulation time can be drastically shortened. For example, the electrophoresis device may be capable of treating 100 samples at a time for 5 hours, preparation of membrane fraction, transfer to liposome and ligand/receptor binding reaction may be all automated and integrated into one device that may be able to simultaneously treat 20 samples in 3 hours. Furthermore, the analysis time by mass spectrometer may be five times shortened.

Approximate time necessary for constructing the database for determining diseases after such shortening is assumed by rough calculation, to analyze 100 cases of each example of 100 disease groups by the above-mentioned method with 10 sets of this device of the invention and 100 sets of mass spectrometer, using serum and one kind of target cell of a disease as a sample, to be 100 × 100 × 3 × 5 / 99 / 10 (152) hours for electrophoresis, 100 × 100 × 2 / 19 / 10 (105) hours for preparation of plate for mass spectrometrys for mass spectrometric analysis, and 100 × 100 × 10.4 × 3 / 100 (3120) for mass spectrometric analysis, thus the rate is defined to be 3120 hours (130 days) of mass spectrometric analysis. Therefore, the database for determining the predetermined 100 diseases can be constructed within half a year, after which integrated diagnoses of 100 diseases in one day becomes affordable. From the foregoing, the impact that this device and this database will have on the medical field is beyond measure.

The ligand sample to be used for this purpose will be body fluid such as blood, serum, urine, cerebro-bone marrow fluid, sperm, saliva, sweat, lacrimal fluid and the like, all intracellular fluid, all intracellular organella fluid and the like. The membrane protein sample is intended to be any cell constituting the body and all organella contained in the cell. From disease taxonomic table of ICD-10, approximately 10,000 diseases are shown. When all organisms are the targets, the number of records of the database constructed using this device is expected to be astronomical figures. However, rapid progress in the information engineering field including hardware and software of computer will permit construction of such exhaustive database. The present invention also represents a tool useful for the development of therapeutic agents of the disease associated with ligand/membrane associated protein. The function analysis of ligand/membrane-associated-protein occupies an important field of biology (inclusive of medicine and agriculture) in the 21st century, and a larger part of the elucidation of the biological function by molecular biology will be clarified in association with some membrane associated protein. While increase and decrease of ligand and increase and decrease of receptor involved with a specific disease can be clarified from the above-mentioned database for diagnosis (the data up to this point is utilized for determination of diagnosis), a further advantage of the present invention rests in the simultaneous determination of counter molecules of both the ligand and membrane protein, meaning specific ligands and specific membrane proteins that bind physiologically. A novel substance in this area has been found by finding one of them (e.g., a ligand) by accident or for some other purpose and then finding the corresponding receptor using the ligand being labeled, or by finding a pair of ligand and receptor by other way round. Until this point, accidental coincidence and long period of patience is required. There are a number of ligands and membrane proteins having unknown functions, and receptors that bind with artificial substances (medicines) but whose physiological ligands are not identified. When plural molecular species (at least two molecular species including one ligand and one corresponding receptor) are acknowledged in somewhere in the ligand-receptor (membrane protein) matrix compartment number obtained by inputting the measurement results of this method and the existent amount increases or decreases disease-specifically, all molecules located at the compartment number can be provided as disease-associated ligand/membrane proteins, and as highly promising candidates for successful studies of drug discovery. For this purpose, a tandem mass spectrometer is used. This enables presumption of the amino acid sequences of all the molecular species that can be ascribed to a specific compartment number.

Because the present method can analyze any membrane protein of a certain cell membrane, related diseases and target cell membrane proteins can be analyzed systematically by one cycle of manipulation, rather than finding each individual membrane protein involved in a disease one by one relying on accidental coincidence.

As shown in Table 2, moreover, when membrane protein of organelle membrane, such as mitochondrial membrane, is focused on, a drug discovery strategy no one would ever have imagined to date, such as classification of diseases, clarification of relationship between diseases, or development of group-specific therapeutic agent, can be afforded based on changes in the ATP producing capability.

**Table 2 Attractive targets for drug discovery in various membranes**

| Membrane | Target |
|---|---|
| Cell membrane | Intracellular signal transduction of extracellular effector molecules |
| Nuclear membrane | DNA replication, RNA transcription/ splicing |
| Mitochodrial membrane | Oxidation-reduction, ATP production |
| Peroxisomal membrane | Peroxide metabolism |
| Lysosomal membrane | Degradation of protein, nucleic acid, saccharide and lipid |
| Golgi apparatus membrane | Transglycosylation and membrane transport |
| Endoplasmic reticulum | Protein/lipid synthesis and membrane transport, MHC |
| X membrane | Reaction site of signal transducer molecule? |

### 7. Other disclosures

The present application also describes measurement principle and method that permit observation and analysis, in an aqueous solution, of an interaction of a molecule insoluble in an aqueous solution and other soluble or insoluble molecules. Therefore, the scope of the present invention is not limited to the finding, quantitation and analysis of membrane protein-ligand complexes derived from the body but goes as far as the finding, quantitation and analysis of an artificially created substances that interact with membrane proteins, finding, quantitation and interaction analysis of two or more membrane proteins capable of interacting with each other, analysis of interaction between insoluble substances other than membrane protein, which is derived from the body, and insoluble or soluble substances, and finding, quantitation and analysis of substances (object) not derived from the body.

Making a sharp contrast to a conventional method of MS analysis of a protein comprising sugar chain and lipid as constituent components, which requires a previous treatment with protease, phospholipase, esterase and the like, a spectrum of a simple protein completely (or partially depending on the conditions) without sugar chain or lipid can be obtained in the present invention by merely changing the composition of the solvent to be added to a sample on a plate for mass spectrometry. The detail is explained in Example 8 below.

The present application further describes an ultrahighly sensitive method for detecting a denatured non-water-soluble protein such as pathogen prion protein and the like. The detail is explained in Example 9 below.

The significance and object of the present invention is not limited to the completion of a full automated proteomic analysis device for membrane protein and ligand, but rests in the technical contribution to the flourishing of the membrane protein science that is responsible for the key part of the life phenomena but fell far behind in the study due to the methodological and technical barriers. As mentioned above, the present invention provides the principle, method and results of grouping proteome into membrane proteins and'water-soluble proteins and embedding the membrane protein in a liposome, which enables handling of the membrane proteins in a similar manner as the handling of water-soluble proteins.

This principle and the method join the new stream of drug discovery explored by the methodology of the genomic drug discovery, and will be certainly recognized in the near future as a membrane protein drug discovery closely related to the technology of receptor related drug discovery, antibody related drug discovery, HTS related drug discovery, protein steric structural analysis, gene expression analysis, other proteome analysis and the like.

The present invention is explained in more detail in the following by referring to Examples that are for exemplification only, and do not limit the present invention in any way.

### Examples

Specific embodiments and results are shown in the following to establish that the method and device of the present invention are effective for the simultaneous screening of the membrane protein and its ligand and analysis of the interaction of the both, and that they are novel method and device for the proteome analysis.

Receptors are classified into three kinds: GPI anchor type, GPCR type and oligomer type, based on the structure. For the present invention to be verified as being useful for the proteomic analysis of membrane proteins, the interaction with each specific ligand should be analyzed with all of these types of receptors as targets. The U937 cell is a cell line derived from human monocyte, and a number of receptors are expressed on its membrane surface. Of these, urokinase receptor was selected as a GPI anchor type, a serum complement component C5a receptor was selected as a GPCR type, and interferon-γ receptor was selected as an oligomer type.

### Reference example 1: Confirmation of expression of three kinds of receptors

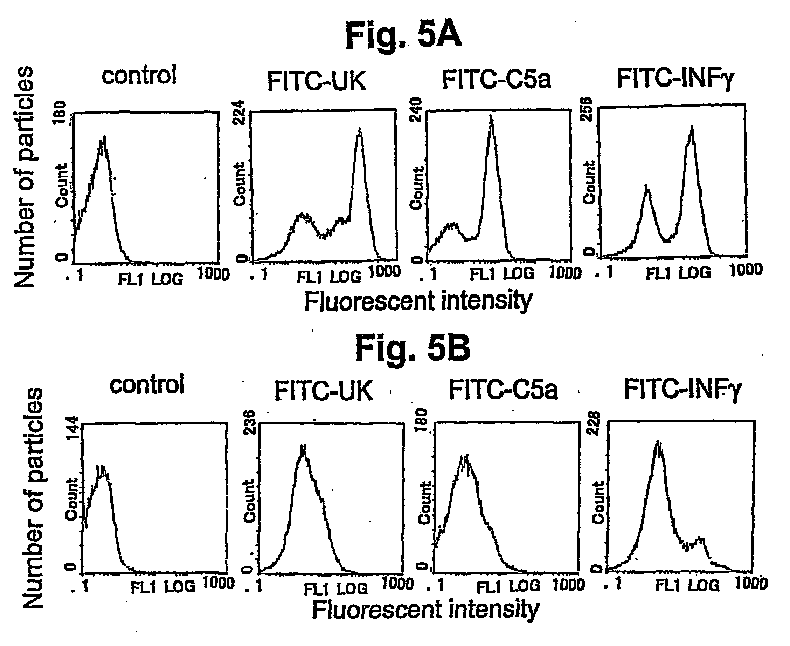

Before and after Bt₂cAMP stimulation, U937 cells were respectively reacted with three kinds of ligands prelabeled with FITC and analyzed by FACS to observe presence of expression of the receptor. As a result, expression of the all three kinds of the receptors was observed as shown in Fig. 5.

### Example 1: Preparation of urokinase receptor-embedded liposome

### (1) Preparation of membrane fraction

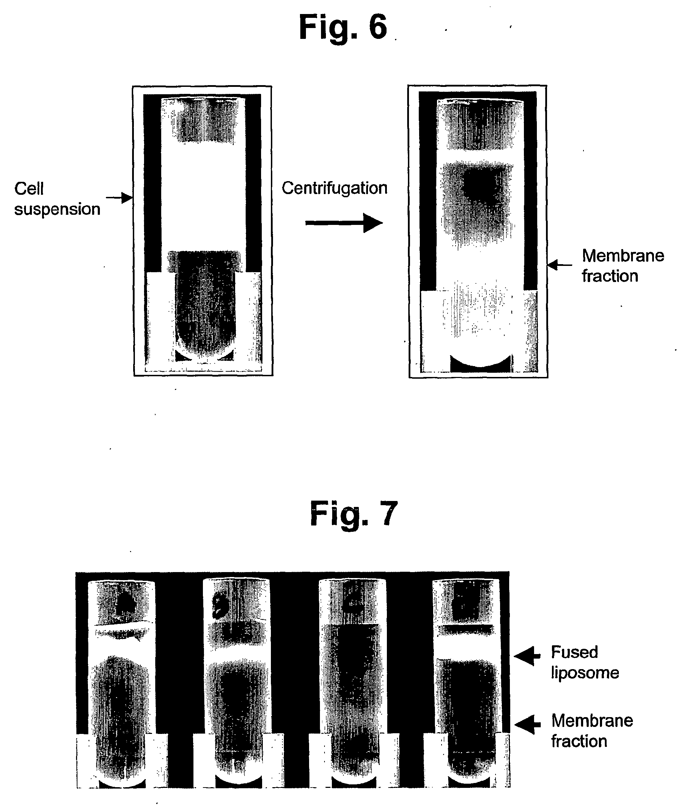

Because U937 is a cell line derived from human monocyte, and expresses a urokinase receptor at high concentration by phorbolester (PMA) stimulation, it was used as a sample for separation of a membrane fraction. After washing, the cells were ruptured by Polytron under ice-cooling for 2-5 sec × 3 times at 1 min intervals, and the membrane fraction was accumulated on the interface by 40% sucrose density gradient centrifugation (95,000 g × 60 min)(Fig. 6).

### (2) Preparation of membrane protein-embedded liposome

Purified yolk lecithin (1.25 g) and cholesterol (0.125 g) were suspended in 25 mL of physiological saline, and treated in a probe type ultrasonication device for 15 min under ice-cooling. The obtained liposome has an average particle diameter of 80 nm. The U937 membrane fraction prepared in advance was added to this liposome solution and freeze and thaw was repeated 3 times at -80°C and room temperature. The mixture became cloudy. When a solution containing liposome lone was treated in the same manner, the liposome solution became cloudy. In contrast, the U937 membrane fraction remained semi-transparent even after repeating freeze and thaw. Cesium chloride was added to these samples and the final concentration was adjusted to 40%, and solutions having a cesium chloride concentration of 30% and 15% and physiological saline were layered in this order on this solution and density gradient centrifugation (95,000 g × 1 h) was performed. As a result, the U937 membrane fraction formed a band in the interface of cesium chloride concentrations of 30% and 15%, and the clouded liposome formed a band in the interface of cesium chloride concentration of 15% and physiological saline. In the mixture of the liposome and the U-937 membrane fraction, a band was observed at the similar position as the clouded liposome and a band was not observed at the position of the U-937 membrane fraction. From the results, the U-937 membrane fraction was assumed to have fused with the liposome and formed a membrane protein-embedded liposome (Fig. 7).

### (3) Confirmation of membrane protein embedded liposome

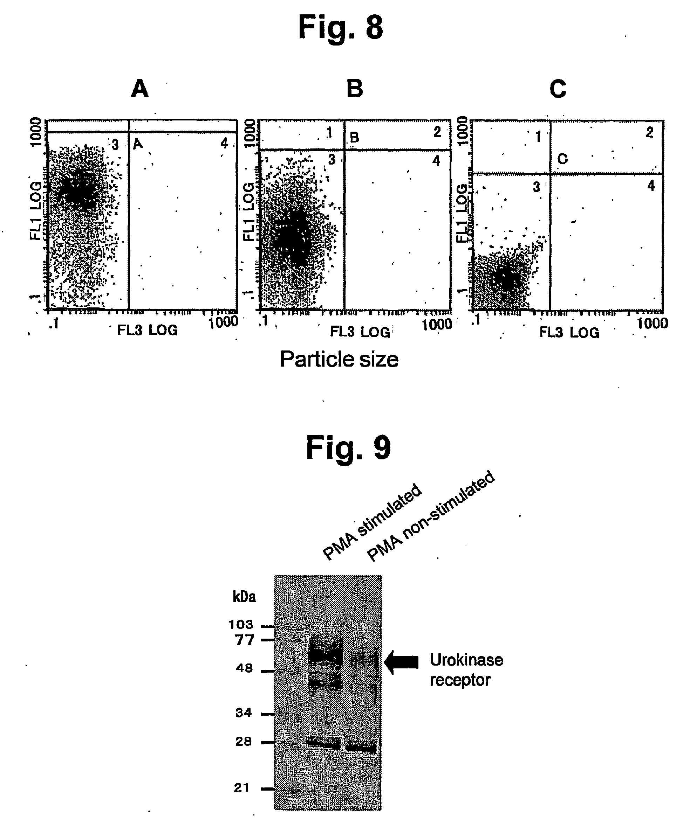

After separation and preparation of the membrane fraction, the membrane protein was fluorescent (FITC) labeled. This membrane fraction was reacted with the liposome by the method of the above-mentioned (2) to form a membrane protein embedded liposome. Each sample of membrane fraction, membrane protein embedded liposome and simple liposome was analyzed by FACS. As a result, as shown in Fig. 8, the fluorescent intensity (y-axis) per particle was the highest for the membrane fraction, the lowest for the simple liposome (with no protein present, weak scattered light is detected rather than FITC fluorescence) and the membrane protein embedded liposome was positioned in between. Since the membrane protein embedded liposome sample hardly contained particles emitting weak scattered light detected in the simple liposome, it was assumed that the membrane fraction and the simple liposome fraction fused almost 100%. That is, the membrane fraction and the simple liposome fused to form a multi-layered liposome, and the membrane protein also transferred to the surface of inner liposomes in the multi-layered liposome. Consequently, the number of membrane protein on the outer membrane surface decreased, which in turn reduced the fluorescent intensity of the membrane protein embedded liposome. From the foregoing, it was shown that the membrane protein bound with the cell and the cell membrane fraction prepared therefrom transferred to the lipid bilayer of the liposome by this preparation method.

### (4) Identification of urokinase receptor (U937 cell)

The U937 cells cultured in the presence or absence of PMA were solubilized, and immunoprecipitated with the goat anti-human urokinase receptor antibody. After electrophoresis, Western blotting was performed and the presence of urokinase receptor on the U937 cell membrane was confirmed. The primary reaction of the Western blotting was a reaction of the biotinylated goat anti-human urokinase receptor antibody at 250-fold dilution for 2 h and the secondary reaction was a reaction with alkali phosphatase-labeled streptoavidin at 100-fold dilution for 1 h. After the reaction, it was washed 3 times with PBS/0.1% Tween 20 and detected by BCIP/NBT. As a result, a broad band of molecular weight 50 kDa stained with an anti-human urokinase receptor antibody was observed, as shown in Fig. 9. Therefrom, the presence of urokinase receptors having different sugar chain structure was found on the U937 cell surface.

### (5) Confirmation of urokinase receptor-embedded liposome

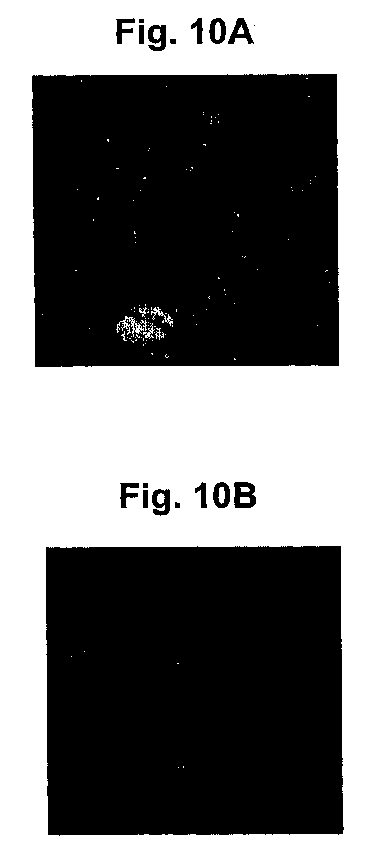

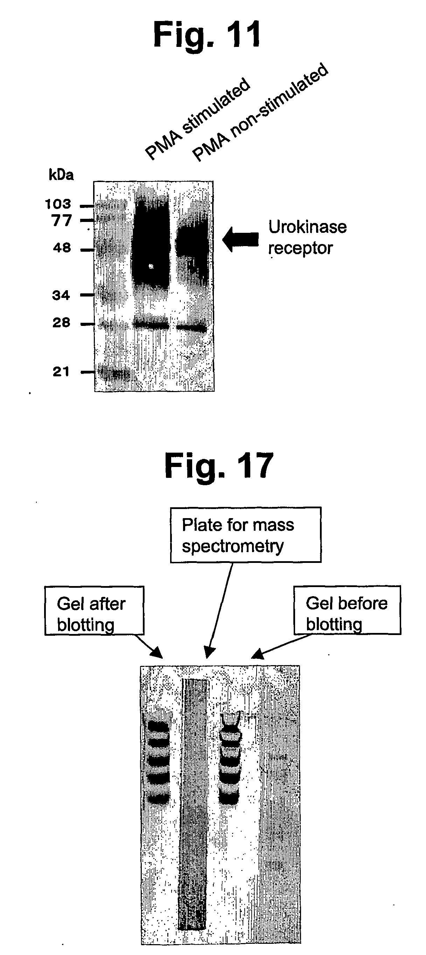

The membrane protein-embedded liposome obtained by the method of the above-mentioned (3) was reacted with a goat anti-human urokinase receptor antibody as a primary antibody at 4°C for 1 h, and reacted with an FITC-labeled rabbit anti-goat IgG antibody as a secondary antibody at 4°C for 1 h. The primary and secondary antibodies were reacted at 10-fold dilution, and after reaction, wash with 0.1% BSA-PBS (containing a protease inhibitor) was performed four times. Then, it was observed by a 320-power confocal scanning laser microscope (LSM410, Carl Zeiss) (Fig. 10). In the fused liposome with the addition of an anti-urokinase receptor specific antibody (Fig.10A), fluorescence was observed but in the fused liposome without the addition of an anti-urokinase receptor specific antibody (Fig.10B), fluorescence was not observed. From the foregoing, it was shown that the urokinase receptor was transferred to the lipid bilayer of the liposome. In the same manner, the obtained membrane protein embedded liposome was solubilized, immunoprecipitated with a goat anti-human urokinase receptor antibody and subjected to Western blotting. The primary reaction of the Western blotting was a reaction with a biotinylated goat anti-human urokinase receptor antibody at 250-fold dilution for 2 h, and the secondary reaction was a reaction with an alkali phosphatase-labeled streptoavidin at 100-fold dilution for 1 h. After the reaction, the sample was washed 3 times with PBS/0.1% Tween 20 and detected by BCIP/NBT. As a result, a broad band of molecular weight 50 kDa stained with an anti-human urokinase receptor antibody was observed, as shown in Fig. 11. The results showed the same molecular weight distribution as that previously confirmed with the U937 cell. Therefrom, it was shown that the urokinase receptor transferred to the lipid bilayer of the membrane protein-embedded liposome retained the sugar chain structure and the like present in the native cell.

### (6) Ligand binding ability of urokinase receptor

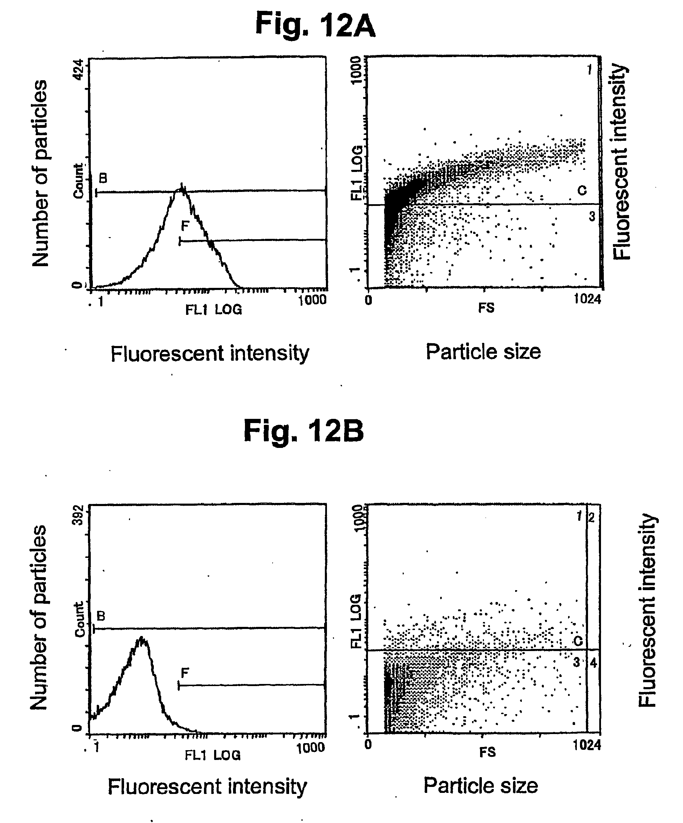

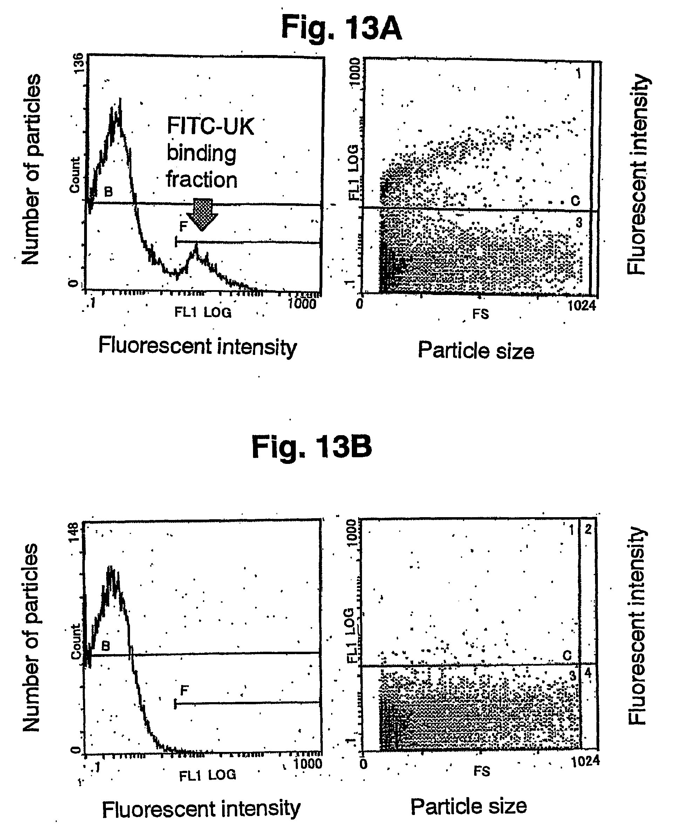

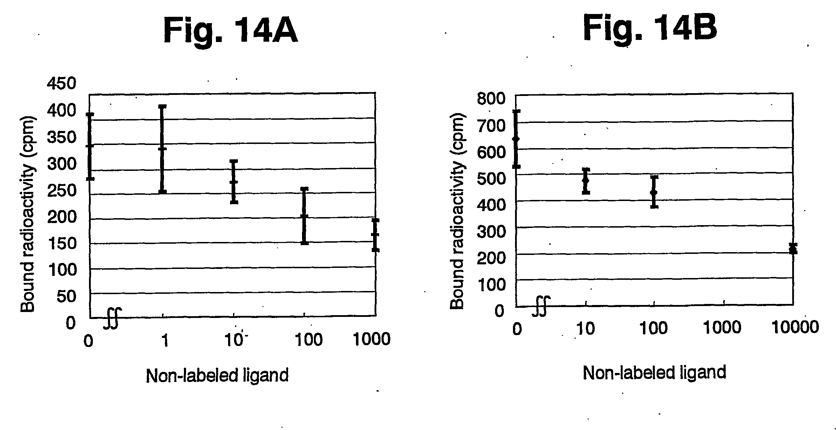

Since the membrane fraction prepared from the U937 cell was shown to have retained a urokinase receptor in the lipid bilayer, the urokinase (ligand) binding ability of the urokinase receptor was studied. Urokinase was fluorescent-labeled by FITC, reacted with a membrane fraction and binding of the receptor and the ligand was examined. As a control, human serum albumin (HSA) fluorescent-labeled by FITC was used. The results are shown in Fig. 12. As is clear from the Figure, the membrane fraction prepared by this method bound with the endogenous ligand urokinase (Fig.12A), but otherwise with HSA (Fig.12B). This means that the urokinase receptor retained in the membrane fraction obtained by this membrane protein preparation method retained the three-dimensional structure and the physiological function (ligand binding ability). Next, the urokinase binding ability of the membrane protein-embedded liposome was examined. Urokinase was fluorescent-labeled by FITC, reacted with the membrane protein embedded liposome (including urokinase receptor embedded liposome) obtained by the earlier method and binding of the receptor and the ligand was examined. As a control, human serum albumin (HSA) fluorescent-labeled by FITC was used. The results are shown in Fig. 13. As is clear from the Figure, the urokinase receptor embedded liposome prepared by this method bound with the endogenous ligand urokinase (Fig.13A) but otherwise with HSA (Fig.13B) To confirm the binding specificity between the urokinase receptor embedded liposome and urokinase, mixtures of an RI-labeled urokinase and a non-labeled urokinase were prepared at various molar ratios of 1:1 - 1:10000, reacted with each of U937 cell and urokinase receptor embedded liposome, and the radioactivities bound with the cell and urokinase receptor embedded liposome were determined. As shown in Fig. 14, the both showed a decrease in the radioactivity bound with the receptor as the non-labeled ligand increased. Therefore, it was postulated that the binding of the urokinase receptor embedded in the liposome and urokinase was specific.

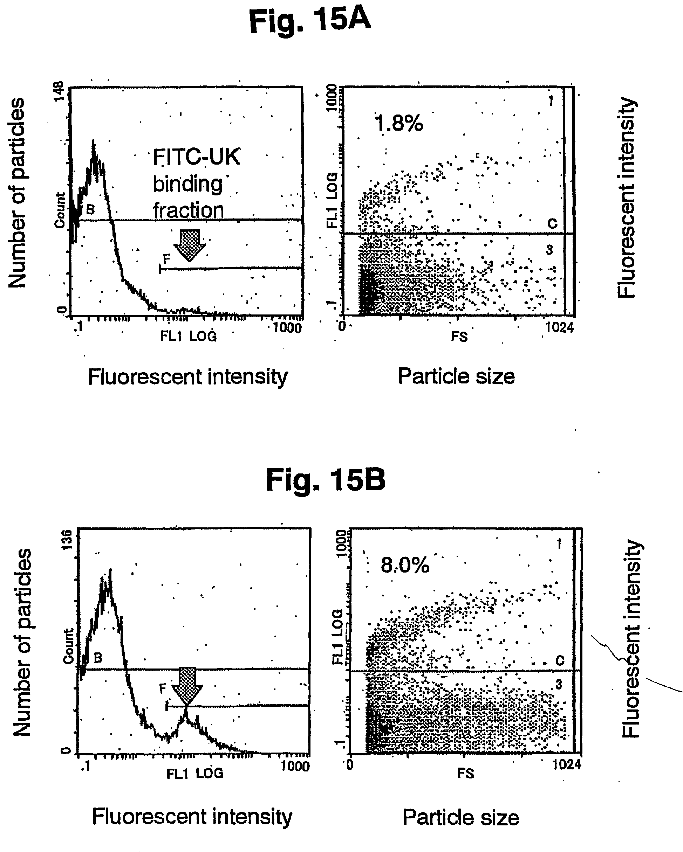

In addition, the change in the expression amount of urokinase receptor by PMA stimulation was studied based on the binding amount of urokinase with the membrane protein embedded liposome. As shown in Fig. 15, the membrane protein-embedded liposome prepared from the PMA stimulated U937 cell clearly showed an increase in the number of liposome particles bound with the fluorescent-labeled urokinase, as compared to those prepared from the untreated cells.

The above results show that the structure and function of the membrane protein embedded in the liposome are the same as those of the membrane protein expressed on the living cell membrane. Therefore, it was shown that the method of the present invention could evaluate the changes in the amount and property of the receptor and ligand during disease, instead of living cells.

### Example 2: Appearance of receptor embedded liposome after decrease in particle diameter

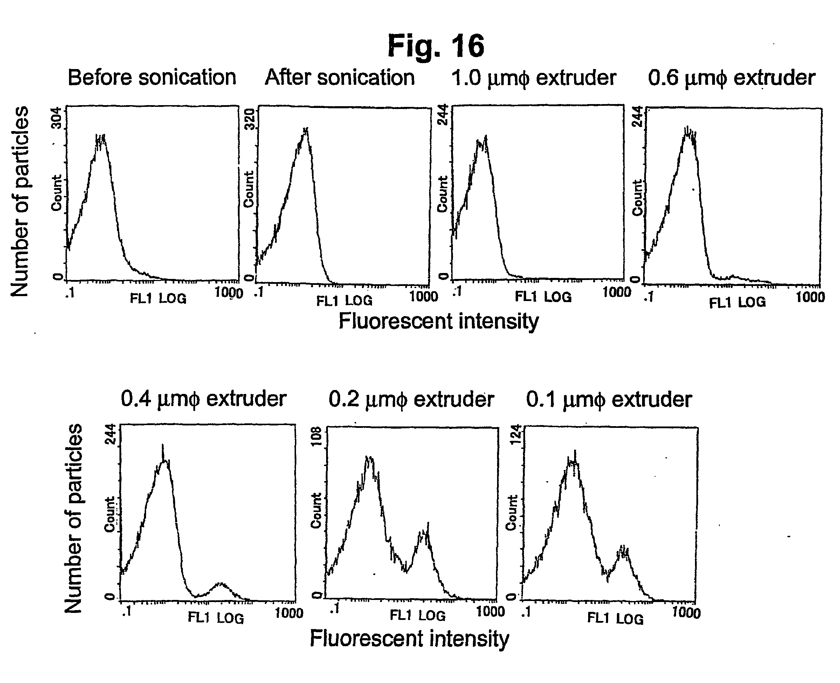

The particle size of the membrane protein-embedded liposome was changed by an extruder method and the appearance of the urokinase receptor embedded liposome was examined with a fluorescent (FITC)-labeled urokinase. As a result, the number of the liposomes with the objective receptor embedded clearly increased in a liposome solution passed through a filter having a filtered pore size of not more than 0.6 µm, as shown in Fig. 16. It is postulated that this was caused by the fact that most of the objective receptors were enclosed inside a large liposome in the multi-layered liposome immediately after fusion and the fluorescence was not detected, and more importantly, a smaller liposome size decreased the number of receptors embedded in one liposome, thereby drawing rigid distinction between the liposomes with the objective receptor embedded and the liposomes without the objective receptor embedded, and the number of the embedded liposomes increased. When the liposomes were sized to a particle size of 10 nm - 5,000 nm, preferably not more than 500 nm, by an extruder method or other methods, a population of the labeled membrane protein-embedded liposome clearly appears in the corresponding size region by FACS analysis.

Therefore, when, for example, cDNA is prepared from the gene sequence of the membrane protein and a host cell is transformed with an expression vector containing said cDNA sequence to allow expression of a membrane protein (incorporating fluorescent molecule and other label molecules recognizable by FACS), and when the non-labeled liposome having the embedded labeled membrane protein prepared from the cell membrane fraction of the expression host according to the method of the present invention is adjusted to the corresponding size and analyzed by FACS, it is obvious that the presence or otherwise of the expression and expression amount of the membrane protein would be detected. In this case, unlike the methods available at present, the detection of the membrane protein under the conditions including no information other than the DNA sequence of the membrane protein, nor a reagent (antibody, ligand, competitive agent, cell responsiveness and other detection reagent of the membrane protein) becomes possible. In addition, if a detection reagent of the membrane protein, such as antibody and the like, is present, labeling at a protein level becomes possible, and so does analysis of expression by FACS. It is needless to say that this method can be applied to the search of ligand based on the similar principles.

### Example 3: Blotting of urokinase onto plate for mass spectrometry

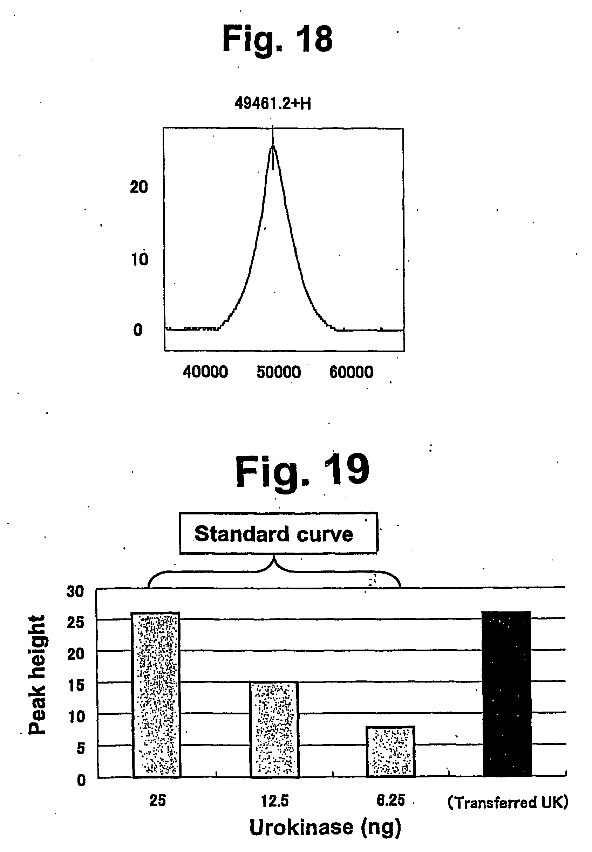

Urokinase (10, 13, 16, 20, 33 µg) was loaded in the same well of polyacrylamide gel at constant time intervals and electrophoresed. After the completion of the migration, the gel was peeled off from the glass plate and, after substituting the solution in the gel with a blotting buffer (5% acetonitrile/125 mM NaCl/PBS) for 5 min, subjected thermal blotting (diffusion blotting) onto an aluminum plate surface-processed with a hydrophobic material having 16 carbons. As the blotting buffer, used was phosphate buffer containing 5% acetonitrile and 125 mM NaCI and blotting was performed at 35°C for 4 h. Fig. 17 is a photograph showing the plate for mass spectrometry after blotting and the gel before and after blotting stained with coomasie brilliant blue. Urokinase band on the plate for mass spectrometry is not visible to the naked eye, but since the urokinase band on the gel is fainter after blotting, a certain amount of urokinase was suggested to have been transferred onto the plate for mass spectrometry.

### Example 4: Identification of urokinase on plate for mass spectrometry by mass spectrometric analysis

After polyacrylamide gel electrophoresis, urokinase transferred onto the plate for mass spectrometry by thermal blotting (diffusion blotting) was directly measured by mass spectrometer, the results of which are shown in Fig. 18. As the conditions of mass spectrometry, Sinapinic acid (saturated solution dissolved in 50% acetonitrile/0.5% trifluoroacetic acid) was used as an energy absorbing substance, which was added by 0.5 µl/spot and air dried, and then the same amount was added and air dried. The measurement was performed by SELDI ProteinChip® System manufactured by Ciphergen Biosystems Inc. The mass calibration was external calibration using bata-lactoglobulin A (bovine), Horseradish peroxidase and conalbumin (chicken). The measurement parameters of SELDI ProteinChip® System were; Detector voltage 2200V, Detector Sensitivity 10, Laser Intensity 285. As a result, one peak was observed at the site of molecular weight 49461.2. Since the urokinase used for examination was precursor and the theoretical molecular weight predicted from the amino acid sequence is 48,525, addition of sugar chain molecule is suggested.

### Example 5: Transfer rate of urokinase onto plate for mass spectrometry

Urokinase transferred onto plate for mass spectrometry was quantitated by mass spectrometric analysis. Using standard urokinase 25, 12.5, 6.25 ng, the standard curve of the peak height obtained by mass spectrometry versus concentration was drawn. Thereafter, urokinase (2,500 ng) was loaded on a polyacrylamide gel and electrophoresed. Urokinase transferred to a plate for mass spectrometry by thermal blotting (diffusion blotting) was directly measured by mass spectrometer. As a result, a peak height corresponding to 25 ng was detected as shown in Fig. 19. From this result, the transfer rate from the gel to the plate for mass spectrometry by thermal blotting method was estimated to be about 1%.

### Example 6: mass spectrometric analysis of bacteriorhodopsin-embedded liposome on a plate for mass spectrometry

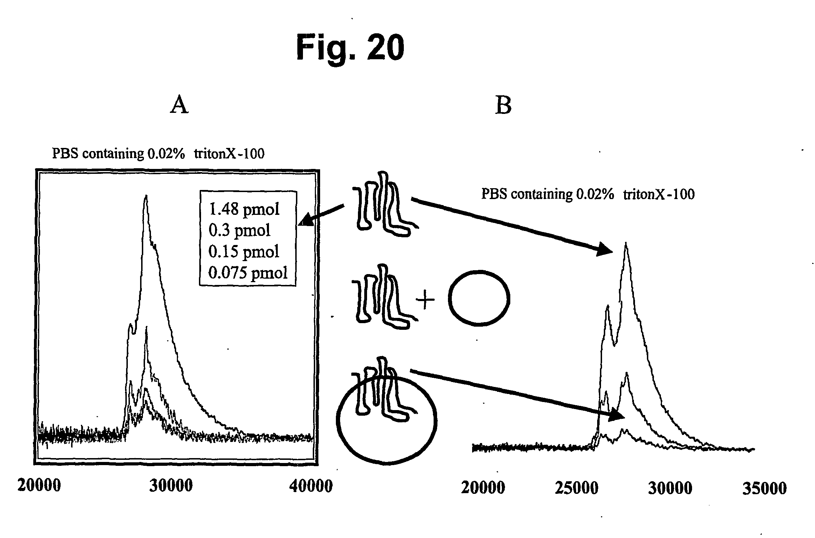

The results obtained by measuring bacteriorhodopsin (Sigma, B3636) using a mass spectrometer are shown in Fig. 20. The sample was put on a chip, air-dried, and measured by SELDI ProteinChip® System (Ciphergen) after addition of 2,5-dihydroxybenzoic acid (170 mg/mL in ethanol) by 0.5 µL/spot and air-dried. The mass calibration was external calibration using bata-lactoglobulin A (bovine), horseradish peroxidase and conalbumin (chicken). The measurement parameters of SELDI ProteinChip® System were Detector voltage 2100 V, Detector Sensitivity 10, Laser Intensity 285. As a result, two peaks at 27027.3 and 28120.2 were observed for every case. From the theoretical molecular weight of 27068.0 of bacteriorhodopsin, the former was bacteriorhodopsin and the latter was suggested to be a bacteriorhodopsin-like protein because it showed retinal elimination upon treatment with an organic solvent.

Fig. 20A shows the correlation between protein concentration and peak intensity when bacteriorhodopsin alone was measured, and from the proportional relationship, the detection limit was 20 fmol. Fig. 20B shows the measurement results of bacteriorhodopsin alone, and bacteriorhodopsin under the conditions of co-existence of a simple liposome and bacteriorhodopsin embedded in a liposome. Under the same solvent condition, these three modes showed different MS signal intensity of bacteriorhodopsin at the same concentration, indicating that the mass spectrometric analysis of bacteriorhodopsin can be carried out even under the conditions of co-existence of a simple liposome and bacteriorhodopsin embedded in a liposome.

### Example 7: mass spectrometric analysis of urokinase receptor on a plate for mass spectrometry

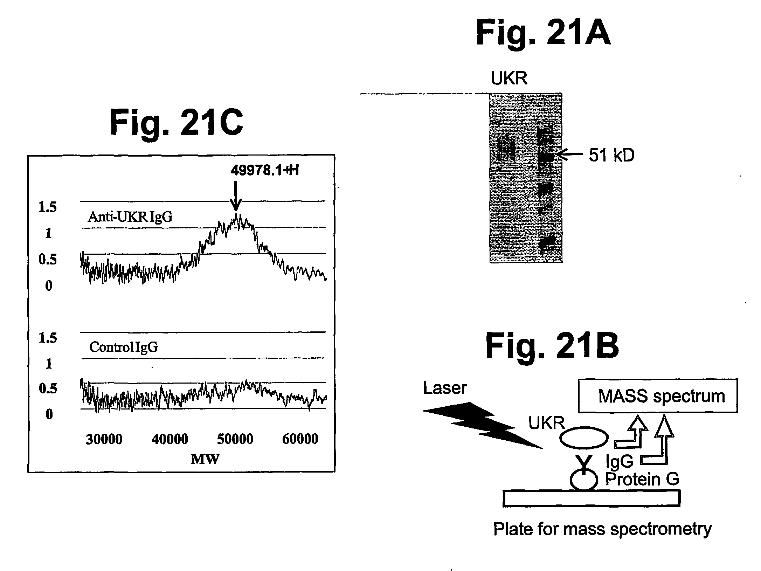

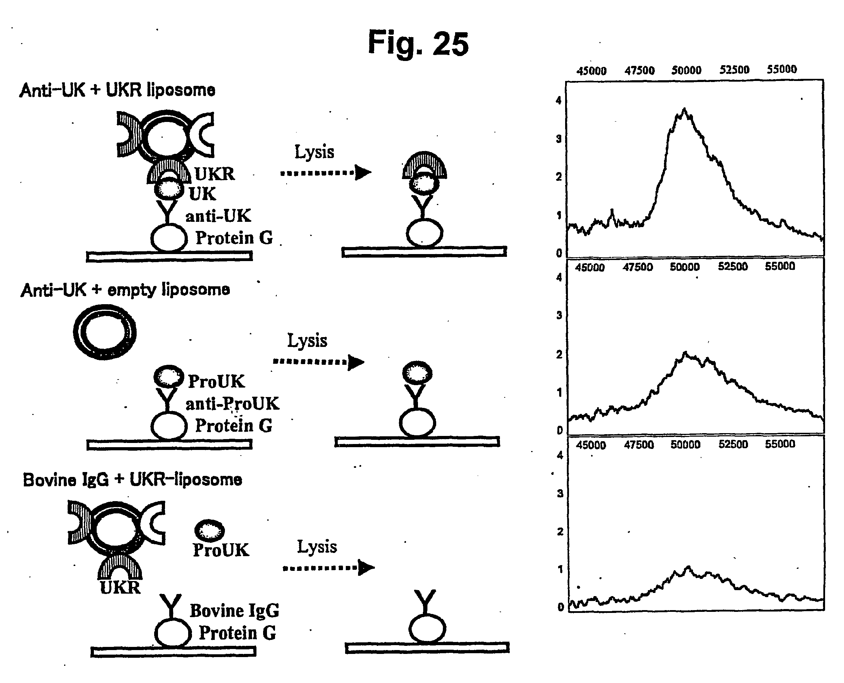

Protein G was crosslinked on a plate for mass spectrometry by a covalent bond, and an anti-urokinase receptor antibody was bound non-covalently to prepare a plate for mass spectrometry. The purification product (Fig. 21A) of urokinase receptor shown in Fig. 21 was applied onto this plate and mass spectrometric analysis was done by a conventional method (Fig.21B). As a result, the peak of the urokinase receptor was found at the position of molecular weight of 49978.1 (Fig. 21C upper) and the anti-urokinase receptor IgG antibody considered to be the ligand of the urokinase receptor was also found in the area of the theoretical molecular weight. When the non-specific IgG was used as a control, mass peak for urokinase receptor was not observed (Fig. 21C lower). Then, U937 cell membrane fraction was subjected to mass spectrometry. As a result, the peak of the urokinase receptor was found at the similar position, supposing the idea that the urokinase receptor from U937 cell membrane fraction can also bind specifically to the anti-urokinase receptor IgG. Additionally, protein G was covalently bound on a plate for the mass spectrometric analysis to bind the anti-urokinase receptor IgG (goat) where the protein G/antibody complex serves as a spacer on the plate. After the purification product of urokinase was applied to the plate, a proteoliposome solution prepared from U937 cell by the conventional method was subjected to the reaction on the plate at 4 °C overnight. The mass spectrometric analysis was performed after the removal of liposome using a buffer containing octylglucoside. As a result, a peak composed of both urokinase receptor and urokinase was found around the region attributable to 50,000 Da (Fig. 25 top). Although a peak was found at the region in the case using a simple liposome without proteins in place of the proteoliposome (Fig. 25 middle), the peak intensity decreased obviously. Namely, the difference between the two peaks originates from the presence or absence of urokinase-receptor, indicating the fact that the urokinase-receptor embedded in the proteoliposome can bind to urokinase bound on the plate. Both the peaks attributable to urokinase receptor and urokinase was not observed in the case using bovine IgG unable to bind urokinase in place of the anti-urokinase IgG (Fig. 25 bottom).

What is to be particularly noted here is the fact that the urokinase receptor embedded in the lipid bilayer was collectively detected and identified with the ligand (urokinase) by mass spectrometry, whereby the basic principle of the present invention was verified.

### Example 8: Removal of sugar component on a plate for mass spectrometry

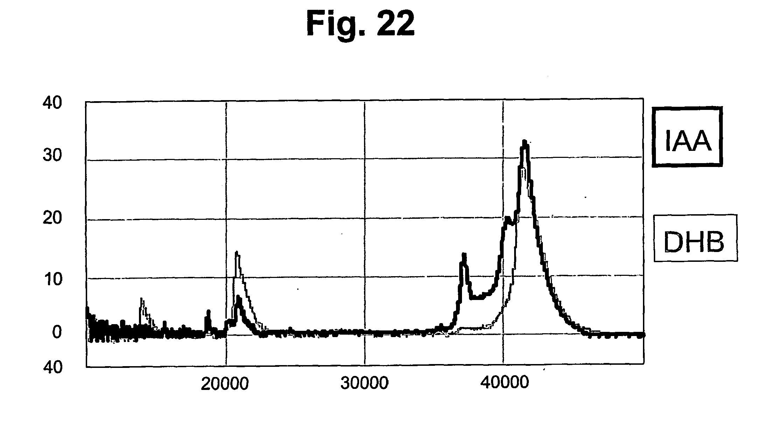

Membrane proteins (urokinase receptor, bovine rhodopsin) and soluble proteins (urokinase, horse radish peroxidase) having sugar chains, and a membrane protein (bacteriorhodopsin) and soluble proteins (β-lactoglobulin, cytochrome c) without a sugar chain were captured on a plate for mass spectrometry, air dried, added with saturated sinpinic acid (SPA), saturated 2,5-dihydroxybenzoic acid (DHB) or indoleacrylic acid (IAA) dissolved in 50% acetonitrile-0.5% trifluoroacetic acid by 0.5 µl/spot and air dried. The measurement was done with a SELDI ProteinChip® System (Ciphergen). The mass calibration was external calibration using ubiquitin (bovine), myoglobin (equine) and serum albumin (bovine). The results are shown in Table 3 and Fig. 22.

**Table 3 Removal of sugar chain on plate for mass spectrometry**

| Protein | | sugar chain | Reduced M.W. |
|---|---|---|---|
| Membrane | UKR | present | ca. 3,000 Da |
| protein | Bacteriorhodopsin | none | 0 Da |
| | Bovine rhodopsin | present | ca. 3,000 Da |
| | ProUK | present | ca. 1,000 Da |
| Soluble | HRP | present | ca. 1,400 Da |
| protein | β-lactoglobulin | none | ca. 45 Da |
| | Cytochrome c | none | ca. 45 Da |

Despite membrane protein or soluble protein, the molecular weights obtained using SPA or IAA are similar to the theoretical value expected from the amino acid sequence while those obtained using DHB were dependent upon the presence of sugar chains of the protein. For example, a protein without sugar chain cytochrome c showed almost the same molecular weight in both cases (12,251 by SPA and 12,297 by DHB), while a protein with sugar chain urokinase receptor showed about 3,000 Da difference between the case using DHB and SPA (47,466 by SPA and 50,465 by DHB). The molecular weights of other proteins with sugar chains obtained using DHB are also 1000 to 3000 Da higher than those obtained using SPA or IAA.

For the mass spectrometric analysis of a protein containing sugar chain or lipid as a component, the method of the present invention enables to obtain the spectrum of the simple protein, removing all (or part, depending on the condition) sugar chain or lipid by merely changing the composition of the solvent to be added to the sample on a plate for mass spectrometry, making a sharp contrast to conventional methods requiring pretreatment with protease, phospholipase, esterase and the like.

The present invention can be applied to any protein, irrespective of whether it is a membrane protein or a water-soluble protein, and the solvent may contain any substance other than the catalyst containing a protein in the composition, i.e., a substance defined to be an enzyme, wherein concentration, order of addition and the like of these and the determination principle, kind and the like of the mass spectrometer are not limited.

### Example 9: Detection of denatured protein by mass spectrometry

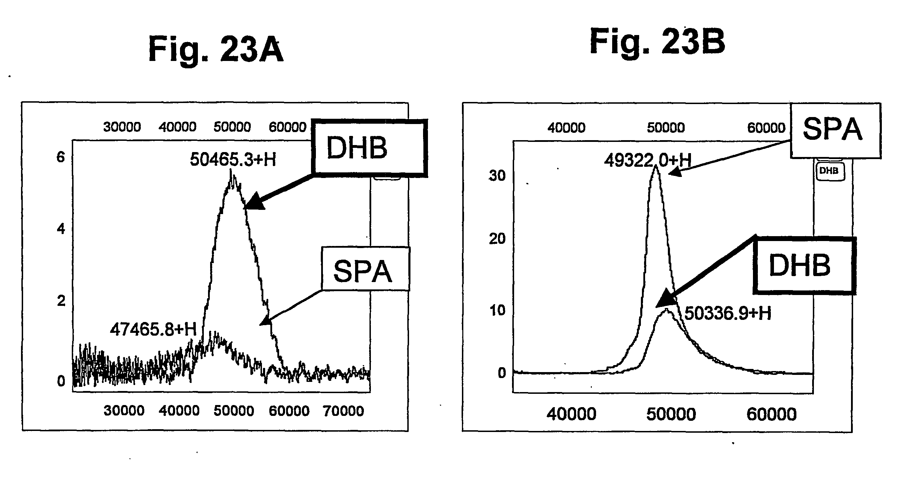

In mass spectrometric analysis, when a water-soluble protein such as cytchrome c, β-lactoglobulin, horseradish peroxidase, urokinase and the like is measured using sinapic acid (SPA) as a solvent, a normal MASS signal intensity appears. When 2,5-dihydroxybenzoic acid (DHB) is used as a solvent, however, the MASS peak became extremely weak (Fig. 23 right). For membrane proteins (bacteriorhodopsin, urokinase receptor), this solvent effect was converse (Fig. 23 left). From this, to detect only a membrane protein group highly sensitively or to detect only a water-soluble protein group highly sensitivity became possible. An important aspect of the present invention is to provide a determination method that affords selective detection of the water-soluble protein and an non-water-soluble protein when a protein, which is soluble under native physiological conditions, is denatured to become an water-insoluble protein for some reason (release of the peptide fragment of itself, genetic mutation, mutual shift to the thermodynamically more stable structure between hetero structure molecules having an identical sequence and the like). This is unattainable by any existing technology.

Therefore, the present invention provides ultrahigh sensitive detection and quantification of pathogenic prion protein (irrespective of biological species of human, sheep, bovine, yeast and the like). This determination principle can be applied not only to other neurodegenerative diseases, such as Alzheimer's disease developed by denatured (insolubilized) amyloid β-protein (Aβ), Familial amyloytic polyneuropathy developed by denatured (insolubilized) transthyretin and the like, but also detection of any disease caused by denatured (insoluble) protein, quarantine of animals, meat inspection and the like. The present invention can be broadly applied to a determination method for sorting denatured condition (insoluble) and physiological condition (soluble) of soluble molecules, such as measurement of renaturation degree of protein in test tubes and the like'.

### Example 10: Binding between ligand bound to a support and membrane protein-embedded liposome

A membrane protein-ligand complex was formed by the use of liposome, biomembrane (membrane fraction) or a biomembrane (cell) as a support for membrane protein, a plate for mass spectrometry, Sepharose 4B gel or magnetic iron particle as a ligand support, and Protein G or biotin as a spacer in combination as shown in Table 4.

**Table 4 Binding between membrane protein embedded liposome and ligand bound with support**

| | Ligand | | Receptor | | Detection method |
|---|---|---|---|---|---|
| Name | Support | Spacer | Name | Support | |
| UK,IF, C5a | None | None | UKR,IFR, C5aR | Liposome | Fluorescence |
| UK | Plate | PoAb /Protein G | UKR | Liposome | Mass spectrometry |
| PoAb | Plate | Protein G | UKR | None | Mass spectrometry |
| PoAb | S4B gel | None | UKR | None | Western blotting |
| UK | S4B gel | None | UKR | Liposome | Fluorescence |
| UK,IF, C5a | S4B gel | Biotin | UKR,IFR, C5aR | Liposome | Fluorescence |
| UK | Magnetic Fe particle | Biotin | UKR | Biomembrane | Fluorescence |

As a result, the presence of a spacer molecule between a support and a ligand was found to be preferable. Depending on the kind of the ligand, when a spacer was not present, the membrane protein-embedded liposome did not bind at all or a binding force was weak. This was because interaction of an avidity effect of multipoint binding was required rather than that of an affinity effect of one point binding, since both molecules were immobilized on a different solid surface. To afford multipoint binding, intervention of a suitable spacer molecule is necessary, which weakens steric hindrance and increases frequency of collision during association of both molecules. A spacer molecule is present between a ligand and a support, consists of a biological polymer (protein etc.), a synthetic polymer, a metal and the like, can be bound by a covalent bond or non-covalent bond depending on the purpose of analysis, and can be a microscopic net shaped, porous shaped and the like having a three-dimensional space.

### Example 11: Binding between ligand immobilized on Sepharose 4B gel and receptor embedded liposome

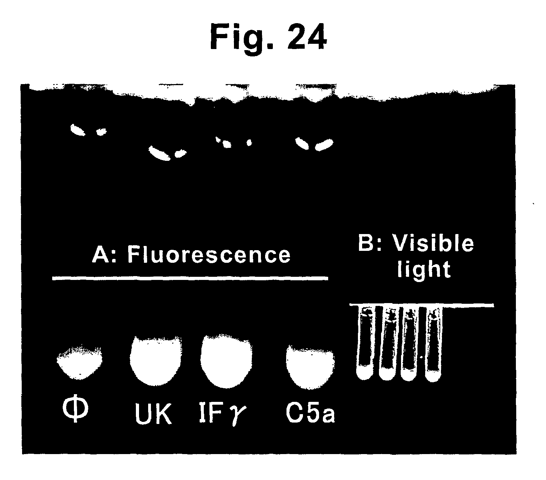

Biotinylated three kinds of ligands (urokinase, interferon-γ, complement C5a) were bound with an avidinylated sepharose 4B gel. The ligand support in this case was Sepharose 4B gel and the spacer was avidin (protein). A membrane protein-embedded liposome was prepared according to the above-mentioned method of Example 1(2) from a fluorescent liposome labeled with FITC and a U937 membrane fraction, reacted with three kinds of ligands immobilized on the sepharose 4B gel, washed three times with PBS, and observed with visible light and fluorescence. As a result, as shown in Fig. 24, all gels were observed white under visible light (B). Under fluorescence (A) in the dark field, color development was not observed in sepharose 4B gel alone, but fluorescence was emitted in other gels using three kinds of ligands bound in combination. This'result reveals that the principle of the present invention can be applied to the combination of any ligand support/any detection system, such as particle/fluorescence, particle/radioactivity, particle/secondary signal generating reagent and the like, besides the aforementioned plate for mass spectrometry/mass spectrometry.

In the above-mentioned examples, detection results of interaction (function) between a urokinase receptor (GPI anchor type), activated complement C5a receptor (GPCR type) and interferon-γ receptor (oligomer type) and their ligands (urokinase, C5a, IFNγ) are shown to establish that the present invention is applicable to any type of receptor. From these results, those of ordinary skill in the art would easily understand that any membrane associated receptor, membrane associated channel, membrane associated pump, membrane associated transporter and a substance concomitantly binds with these proteins can be isolated and identified with ligand, irrespective of the molecular'structure and function, according to the principle and method of the present invention.

While the basic specification and examination results of the respective components of the full automatic proteome analysis device for membrane protein and ligand in the case of mass spectrometry as the measurement method are shown, those of ordinary skill in the art would easily understand that the present invention can be applied to a broad array of uses, as indicated by the examples using a fluorescent-labeled liposome and a ligand-particle conjugate assuming fluorescence analysis.

## Claims

1. A proteome analysis method comprising analyzing, based on biological affinity, both membrane proteins and water-soluble proteins capable of interacting with the membrane proteins, wherein the method comprises the step of:
(a) isolating a water-soluble protein fraction from a biological sample, separating water-soluble proteins in the fraction by gel electrophoresis, bringing the gel after electrophoresis into contact with a ligand support having a surface that can immobilize the proteins retaining the physiological function, and transferring the water-soluble proteins in the gel onto the ligand support;
(b) isolating a membrane fraction from a cell sample and fusing the membrane fraction with liposomes to prepare a membrane protein library wherein all membrane proteins are attached to or penetrated into its lipid bilayer;
(c) bringing the water-soluble proteins immobilized on the ligand support in contact with the membrane protein library to trap membrane proteins having affinity to the water-soluble proteins on the ligand support; and
(d) analyzing both or either of the membrane proteins and the water-soluble proteins having affinity by a means capable of analyzing at least one of the physical or chemical properties of those proteins.

2. The method of claim 1, wherein the means is selected from the group consisting of mass spectrometry, fluorescent method, RI method and surface plasmon resonance method.

3. The method of claim 1, wherein the support is selected from the group consisting of a plate and particles.

4. A method for identifying membrane proteins and/or water-soluble proteins capable of interacting with the membrane proteins that show disease-specific changes in amount or property, which method comprises subjecting a sample collected from an organism suffering from a certain disease to the proteome analysis method of any of claims 1 to 3, and comparing the obtained analysis data with the data of a healthy homologous organism.

5. A method for constructing a database, which comprises applying the method of claim 4 with regard to at least one disease, and pooling the obtained data.

6. The method of claim 4, wherein the proteome analysis is performed according to the method of any one of claims 1 to 3.

## Patentansprüche

1. Proteom-Analyseverfahren umfassend das Analysieren, basierend auf biologischer Affinität, sowohl von Membranproteinen als auch von wasserlöslichen Proteinen, die zur Wechselwirkung mit den Membranproteinen fähig sind, wobei das Verfahren die Schritte umfasst:
(a) das Isolieren einer wasserlöslichen Proteinfraktion aus einer biologischen Probe, das Trennen der wasserlöslichen Proteine in der Fraktion durch Gelelektrophorese, das Inkontaktbringen des Gels nach der Elektrophorese mit einem Liganden-Träger mit einer Oberfläche, die die Proteine unter Aufrechterhalten der physiologischen Funktion immobilisieren kann, und das Übertragen der wasserlöslichen Proteine in dem Gel auf den Liganden-Träger;
(b) das Isolieren einer Membranfraktion aus einer Zellprobe und das Fusionieren der Membranfraktion mit Liposomen, um eine Membranprotein-Bibliothek herzustellen, wobei alle Membranproteine angeheftet sind an oder eingedrungen sind in ihre Lipiddoppelschicht;
(c) das Inkontaktbringen der wasserlöslichen Proteine, die auf dem Liganden-Träger immobilisiert sind, mit der Membranprotein-Bibliothek, um Membranproteine abzufangen, die eine Affinität zu den wasserlöslichen Proteinen auf dem Liganden-Träger besitzen; und
(d) das Analysieren sowohl der Membranproteine als auch der wasserlöslichen Proteine, die Affinität haben, oder von einem von beiden durch Mittel, die geeignet sind, mindestens eine der physikalischen oder chemischen Eigenschaften dieser Proteine zu analysieren.

2. Verfahren gemäß Anspruch 1, wobei die Mittel ausgewählt sind aus der Gruppe bestehend aus Massenspektrometrie, einer Fluoreszenz-Methode, einer RI-Methode und einer Oberflächenplasmonresonanz-Methode.

3. Verfahren gemäß Anspruch 1, wobei der Träger ausgewählt ist aus der Gruppe bestehend aus einer Platte und Partikeln.

4. Verfahren zum Identifizieren von Membranproteinen und/oder wasserlöslichen Proteinen, die fähig sind, mit den Membranproteinen in Wechselwirkung zu treten, welche erkrankungsspezifische Veränderungen in der Menge oder Eigenschaft zeigen, wobei das Verfahren die Durchführung des Proteom-Analyseverfahrens gemäß einem der Ansprüche 1 bis 3 mit einer Probe umfasst, welche aus einem Organismus, der an einer bestimmten Erkrankung leidet, gewonnen wurde, und das Vergleichen der erhaltenen Analysedaten mit den Daten eines gesunden, homologen Organismus.

5. Verfahren zum Aufbauen einer Datenbank, welches die Anwendung des Verfahrens gemäß Anspruch 4 im Hinblick auf mindestens eine Erkrankung und das Zusammenfassen der erhaltenen Daten umfasst.

6. Verfahren gemäß Anspruch 4, wobei die Proteomanalyse nach dem Verfahren gemäß einem der Ansprüche 1 bis 3 ausgeführt wird.

## Revendications

1. Procédé d'analyse de protéome, comprenant l'analyse, basée sur l'affinité biologique, à la fois de protéines membranaires et de protéines hydrosolubles capables d'interagir avec les protéines membranaires, dans lequel le procédé comprend les étapes suivantes :
(a) isolement d'une fraction protéique hydrosoluble à partir d'un échantillon biologique, séparation des protéines hydrosolubles dans la fraction par électrophorèse en gel, mise en contact du gel après électrophorèse avec un support ligand ayant une surface capable d'immobiliser les protéines conservant leur fonction physiologique, et transfert des protéines hydrosolubles dans le gel sur le support ligand ;
(b) isolement d'une fraction membranaire à partir d'un échantillon de cellules et fusion de la fraction membranaire avec des liposomes pour la préparation d'une banque de protéines membranaires dans laquelle toutes les protéines membranaires sont attachées à ou introduites dans sa bicouche lipidique ;
(c) mise en contact des protéines hydrosolubles immobilisées sur le support ligand, avec la banque de protéines membranaires pour piéger les protéines membranaires ayant une affinité avec les protéines hydrosolubles sur le support ligand ; et
(d) analyse à la fois des protéines membranaires et des protéines hydrosolubles ou soit des protéines membranaires, soit des protéines hydrosolubles ayant une affinité, par un moyen capable d'analyser au moins l'une des propriétés physiques ou chimiques de ces protéines.

2. Procédé selon la revendication 1, dans lequel les moyens sont choisi dans le groupe constitué par la spectrométrie de masse, la méthode fluorescente, la méthode IR et la méthode de résonance de plasmon de surface.

3. Procédé selon la revendication 1, dans lequel le support est choisi dans le groupe constitué par une plaque et des particules.

4. Procédé pour l'identification de protéines membranaires et/ou de protéines hydrosolubles capables d'interagir avec les protéines membranaires qui manifestent des altérations, en quantité ou en propriétés, spécifiques de maladies, lequel procédé comprend le fait de soumettre un échantillon, recueilli à partir d'un organisme souffrant d'une certaine maladie, au processus d'analyse de protéome de l'une quelconque des revendications 1 à 3, et la comparaison des données analytiques obtenues avec les données d'un organisme homologue sain.

5. Procédé pour l'établissement d'une base de données, comprenant l'application du procédé selon la revendication 4 eu égard à au moins une maladie, et la réunion des données obtenues.

6. Procédé selon la revendication 4, dans lequel l'analyse de protéome est exécutée conformément à l'une quelconque des revendications 1 à 3.
